# EUROPEAN PATENT SPECIFICATION

(11) **EP 2 536 396 B1**
(45) Date of publication and mention of the grant of the patent: **10.08.2016**
(21) Application number: 11709813.7
(22) Date of filing: 17.02.2011
(51) Int. Cl.: A61K 9/16, A61K 9/20, A61K 31/41

(54) **PROCESS FOR THE PREPARATION OF ORAL SOLID DOSAGE FORMS COMPRISING VALSARTAN**
VERFAHREN ZUR HERSTELLUNG VON ORALEN FESTEN DARREICHUNGSFORMEN MIT VALSARTAN
PROCÉDÉ POUR LA PRÉPARATION DE FORMES POSOLOGIQUES SOLIDES ORALES COMPRENANT DU VALSARTAN

(30) Priority: 16.02.2010 SI 201000060
(43) Date of publication of application: 26.12.2012
(73) Proprietor: KRKA, D.D., Novo Mesto, 8501 Novo mesto (SI); UAB Krka Lietuva, 14013 Vilniaus r. (LT)
(72) Inventor: VRBINC, Miha, 8000 Novo mesto (SI); OSOLNIK, Renata, 8351 Straza (SI); VRECER, Franc, 8351 Straza (SI); ZIBERT, Tanja, 1360 Vrhnika (SI); BUKOVEC, Polona, 8000 Novo mesto (SI)
(74) Representative: Andrae | Westendorp Patentanwälte Partnerschaft
(86) International application number: PCT/LT2011/000002
(87) International publication number: WO 2011/102702

(56) References cited:
- WO-A1-2008/044862
- WO-A1-2009/022169

## Description

### Field of the invention

The invention relates to a process for the preparation of a solid pharmaceutical composition comprising valsartan and the valsartan comprising final oral dosage form as defined by the claims.

### Background of the Invention

Valsartan of formula (I) with its chemical name *N*-(1-oxopentyl)-*N*-{4-[2-(1H-tetrazole-5-yl)phenyl]-benzyl}-L-valine inhibits the angiotensin-converting enzyme and is widely used for the treatment of hypertension and related diseases and conditions.

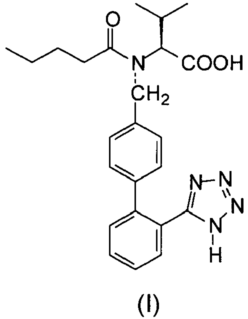

In the context of this invention, the term "valsartan" also includes pharmaceutically acceptable salts, hydrates and solvates of a compound having formula (I).

EP 443983 describes a pharmaceutical composition of valsartan and a process for its preparation. Valsartan, lactose and maize starch are mixed and granulated with an ethanolic dispersion of gelatine. After drying, the rest of maize starch, talc, magnesium stearate and colloidal silica (highly dispersed) are admixed to the granulate and compressed into tablets. Tablets can be film-coated. In another embodiment valsartan, lactose and maize starch are mixed and granulated with a dispersion of maize starch in warmed water. After drying, the rest of maize starch, talc and calcium stearate are admixed to the granulate and compressed in cores. Cores are coated with a dispersion of hydroxypropyl methylcellulose and shellack in dichloromethane. The patent family of EP 914119 discloses a dry granulation technological procedure which comprises the following steps:
- grinding valsartan and pharmaceutically acceptable additives,
- subjecting a mixture of the ground active agent and additives to compression to form a comprimate (the compacted mass),
- converting the comprimate to form a granulate and
- compressing the granulate to form the solid oral dosage form.

The process is carried out in the absence of water, i.e. it is a dry granulation/compaction method. WO 00/38676 describes solid oral dosage forms comprising valsartan (20 to 65%), microcrystalline cellulose (31 to 65%) and crospovidone (2 to 13%). WO 01/97805 claims a composition of valsartan or a pharmaceutically acceptable salt or hydrate thereof and a disintegrant in a weight ratio of between 5.1:1 and 0.5:1. WO2008/006716 discloses a process which involves the steps of: a) providing a mixture comprising: a water-insoluble sartan, a water soluble carrier, a solvent for each of the sartan and the carrier, and b) spray-drying the mixture to remove the solvent or each solvent and obtain a substantially solvent-free nano-dispersion of the sartan in the carrier. WO2007/077581 discloses solid oral dosage forms of hydrophobic active ingredients prepared by treating pharmaceutically effective amounts of the active ingredient with a particle separating agent. This application further discloses solid oral dosage forms of hydrophobic actives without or with a minimum amount of disintegrants. WO2008/056375 and WO2007/052307 discloses a pharmaceutical composition of valsartan prepared by wet granulation wherein the active agent is present in an amount less than 35% by weight based on the total weight of the pharmaceutical composition. WO 2009/022169 A1 relates to stable solid pharmaceutical compositions comprising valsartan as the active pharmaceutical ingredient. Optionally the compositions comprise one or more further active pharmaceutical ingredients. WO 2009/022169 A1 further relates to methods for preparing said compositions and to the use of said compositions in the treatment or prevention of angiotensin receptor mediated disorders, in particular hypertension and related disorders. WO 2008/044862 A1 relates to a functional combination preparation comprising a dihydropyridine-based calcium channel blocker such as amlodipine and an ARB (Angiotensin-2 receptor blocker) such as losartan. In particular, WO 2008/044862 A1 relates to a chronotherapeutical combination pharmaceutical formulation with controlled-release for the prevention or treatment of cardiovascular disease, which is formulated in accordance with xenobiotics and chronotherapy for enabling the two drugs to be chronotherapeutically released. WO03/097045 discloses a pharmaceutical composition containing an angiotensin receptor blocker, a calcium channel blocker and a diuretic. There exists a need in the art for a wet granulation process for the preparation of a solid pharmaceutical composition comprising valsartan including an efficient granulate drying step.

### Summary of the invention

The problem described above was efficiently solved by our finding that using specific conditions and drying regime for the granulate in the wet granulation process provided a granulate with a higher specific surface area and, in spite of the higher specific surface area of the granulate, higher purity and resistance to heat and moisture. The valsartan granulate thus obtained is incorporated into a final oral dosage form comprising valsartan with higher purity.

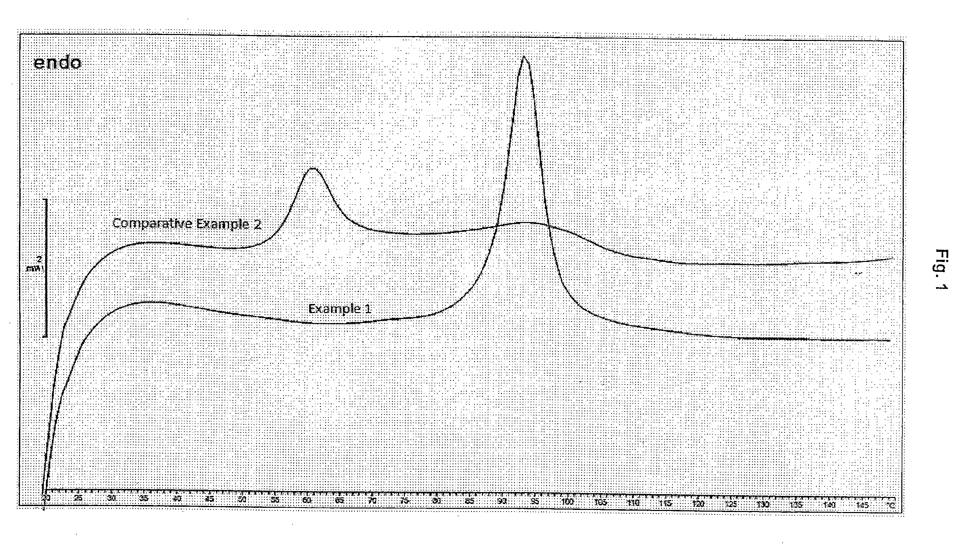

If the drying process of the granulate is performed according to the invention the melting peak of valsartan is detected in the range 90-100 °C in the DSC curve and there are no other peaks and anomalities in the DSC curve in the investigated temperature range, as can be seen from Figure 1 under "Example 1".

If the drying process of the granulate is not performed according to the invention a new peak in the DSC curve appears at around 60 °C, the melting peak of valsartan is shifted towards lower temperatures and becomes broader, as can be seen from Figure 1 under "Comparative Example 2".

### Description of the figures

Fig. 1 shows a DSC thermogram of a valsartan comprising tablet with the characteristic peaks appearing after unsuitable drying of the granulate and characteristic peaks appearing after the drying of valsartan granulate according to the invention.

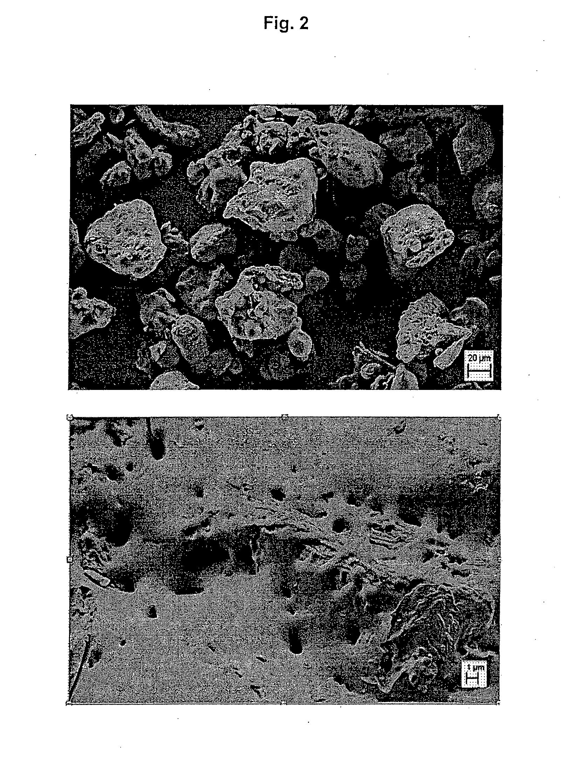

Fig. 2 shows microphotographs of the surface of valsartan comprising granulate, prepared according to Comparative Example 2 and exposed to 40°C and 75 % rel. humidity for 24 hours (magnification 1000x and 10.000x).

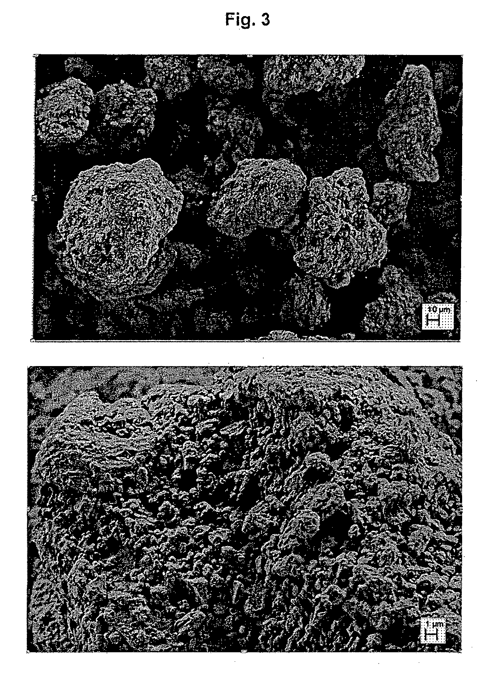

Fig. 3 shows microphotographs of the surface of valsartan comprising granulate, prepared according to Example 1 and exposed to 40°C and 75 % rel. humidity for 24 hours (magnification 1000x and 10.000x)

### Detailed description of the invention

The solid pharmaceutical compositions prepared according to the present invention comprises valsartan in particulate (granulated) form as described in WO2006/066961. In another aspect of the invention, the solid pharmaceutical composition of the present invention further comprises at least one active ingredient selected from diuretics, antihypertensives, lipid regulators and antidiabetics in the form of free acid, free base, salt or ester, wherein further active ingredient can be incorporated into solid composition intragranularly together with valsartan or extragranularly wherein further active ingredient can be formulated into separate granulate or admixed with at least one excipient and valsartan and compressed into tablets. In an another aspect of present invention at least one further active ingredient can be formulated in solid composition containing valsartan in a form of a coating obtained either by compression coating or by coating the valsartan containing core with a coating dispersion containing dissolved, suspended or emulsified further active ingredient and at least one further excipient, or by powder coating, where further drug is in powder form optionally admixed with at least one further excipient and the coating is performed by adhesion of powder mixture on the cores containing valsartan by spraying binder solution, which can optionally contain further excipients.

In the present invention the valsartan comprising tablet core can also be compression coated with a granulate comprising at least one further active substance selected from calcium channel blockers such as amlodipine or its salts, and/or diuretic such as hydrochlorothiazide and/or indapamide, and/or holesterol lowering agent such as HMG-CoA reductase inhibitors, such as rosuvastatin, simvastatin, lovastatin, atorvastatin, fibrates, bile acid sequestrants and/or nicotinic acid. The compression coated solid composition according to present invention can be optionally film coated as described previously.
In a special embodiment of present invention further active ingredient is selected from amlodipine or its pharmaceutically acceptable salts. Dual, triple or higher combinations where valsartan is combined with one or two or more further active ingredients in single solid dosage forms are also included. In yet another aspect of the invention, the solid pharmaceutical valsartan composition of the present invention also comprises a diuretic, preferably hydrochlorothiazide in particulate form, or amlodipine and a diuretic, preferably amlodipine and hydrochlorothiazide.

### Preparation process

The present pharmaceutical formulations are prepared by known technological procedures, wet granulation, preferably aqueous granulation, using well known and readily available excipients. In the preparation of the compositions of valsartan, the active ingredients will usually be mixed with an excipient or mixture of excipients, or diluted with an excipient or a mixture of excipients, or enclosed within an excipient or mixture of excipients which may be in the form of a tablet, microtablet, capsule, sacchet and/or pellet. We have surprisingly found out that when using the wet granulation process drying of the granulate in a specific manner, the drying comprising two steps, both at low absolute inlet air humidity (<3 g water /kg of air), wherein the first step comprises drying at a temperature of 0°C to 40°C for a period of 1 to 7 hours, and the second step comprises drying at a temperature of 40°C to 80°C for a period of 1 to 60 minutes, the granulate comprises less oxidation and degradation products and higher specific surfaces of the granulate when compared to granulates and final oral dosage forms prepared according to other technological processes. This increase in the specific surface of the granulate surprisingly leads to a granulate with a higher purity and increased resistance to heat and moisture and consequently to improved purity and increased resistance to heat and moisture of the final oral dosage forms incorporating such a valsartan granulate.
If the drying process of the granulate is performed according to the invention the melting peak of valsartan is detected in the range 90-100 °C in the DSC curve and there are no other peaks and anomalities in the DSC curve in the investigated temperature range, as can be seen from Figure 1.

If the drying process of the granulate is not performed according to the invention a new peak in the DSC curve appears at around 60 °C, the melting peak of valsartan is shifted towards lower temperatures and broadens.

In the wet aqueous granulation process, the fluid-bed dryer enables the preparation of round-shaped particles of granulate consisting of valsartan and at least one excipient, which provides a reproducible and processable formulation. This can otherwise be a problem due the high weight % of valsartan (>20%) in the solid oral dosage form.

The wetting of a mixture of valsartan and excipient(s) can be performed in conventional granulation equipment by spraying organic solvent, a mixture of organic solvent and water in any ratio, water or an aqueous granulating liquid onto an excipient or mixture of excipients and/or active substances by conventional pharmaceutical techniques. Organic solvent is preferably selected form pharmaceutically acceptable alcohols. Wetting can be also affected by direct addition of water or and aqueous granulating liquid to a mixture of excipients during a mixing operation in a proper mixing device, e.g. a high-shear mixer. The term "aqueous granulating liquid" refers to an aqueous dispersion which comprises purified or demineralised water as a vehicle and a solid substance which is dispersed in the vehicle. The dispersed substance can have known functions of excipients such as surfactant, soluble diluent, stabilizer, pH modifier, suspending agent or binding agent, preferably binding agent, i.e. a binder.

The mixing of excipients or of excipients with valsartan may be effected in conventional devices used for the mixing of powders, e.g. motionless (passive) mixers, fluidized bed, diffusion, biconic diffusion, biconic, turbular, cubic, planetary, Y-, V-shaped or high-shear mixers.

For the granulation, conventional drying devices such as a fluid-bed dryer or drying chambers with controlled inlet air temperature and absolute inlet air humidity can be used. In a special embodiment drying can be performed or facilitated by the use of reduced pressure in the drying chamber or microwaves or by the combination of heat and vacuum (reduced pressure) or combination of microwaves and vacuum.

In the processes according to the invention as described above, the compression, in particular to tablets, can be effected using an automatic rotary compressing machine from different manufacturers of equipment for use in pharmaceutical industry.

Conventional equipment can be used for applying a film coating, such as fluid bed coating system or conventional coating pans for use in pharmaceutical industry.

Valsartan is first prepared according to a suitable synthetic process and then purified, e.g. by crystallization or any other means known to the person skilled in the art. The size of the valsartan particles is as according to WO2006/066961.

In a preferred embodiment, the wet aqueous granulation process comprises:
(i) preparing a compression mixture by using using water or water-based dispersion as granulating liquid to obtain a granulate by
   o (a1) granulating a mixture of one or more excipients and the granulating liquid to obtain a granulate, (a2) drying at absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (a3) adding of said valsartan, one or more further pharmaceutically active compounds and optionally further excipient(s) to the granulate to obtain a compression mixture;
   o (b1) granulating a mixture of one or more excipients, valsartan and the granulating liquid to obtain a granulate, (b2) drying at an absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (b3) adding of one or more further pharmaceutically active compounds and optionally further excipient(s) to the granulate to obtain a compression mixture;
   o (c1) granulating a mixture of one or more excipients, a portion of valsartan and the granulating liquid to obtain a granulate, (c2) drying at an absolute inlet air humidity of less than 3 g water /kg of air in two steps, wherein the first step comprises drying at a temperature a temperature of 0°C to 40°C for a period of 1 to 7 hours, and the second step comprises drying at a temperature of 40°C to 80°C for a period of 1 to 60 minutes, (c3) adding of the rest of the said valsartan, one or more further pharmaceutically active compounds and optionally further excipient(s) to the granulate to obtain a compression mixture;
(ii) compressing the compression mixture to the desired form, and
(iii) optionally applying a coating.

In this process, the step (i) includes three alternatives. In the first alternative (steps a1 to a3) all valsartan is added extragranularly, in the second alternative (steps b1 to b3) all valsartan is included into the granulate (intragranularly), and in the third alternative (steps c1 to c3) valsartan is divided between the intragranular and extragranular phase.

In another embodiment of the present invention, amlodipine or its pharmaceutically acceptable salts can be included into the present valsartan formulations. Depending on the alternative chosen for step (i) further active ingredients selected from the list defined below such as but not limited to amlodipine or its pharmaceutically acceptable salts can be optionally added to any of the steps (a1) to (a3), (b1) to (b3), and (c1) to (c3). Hence, amlodipine or its pharmaceutically acceptable salts, if added, can be present exclusively within the granulate, exclusively as extragranular phase after the granulation, or divided between the granulate and the extragranular phase, independently from the distribution of the valsartan achieved by selecting the desired alternative (a1 to a3), (b1 to b3) or (c1 to c3) of step (i).

In a further embodiment of the invention, hydrochlorothiazide can also be included in the present valsartan or valsartan/amlodipine formulations. Depending on the alternative chosen for step (i), in the present wet granulation process hydrochlorothiazide can be optionally added to any of the steps (a1) to (a3), (b1) to (b3), and (c1) to (c3). Hence, hydrochlorothiazide, if added, can be present exclusively within the granulate, exclusively as the extragranular phase after the granulation, or divided between the granulate and the extragranular phase, independently form the distribution of the valsartan or valsartan and amlodipine achieved by selecting the desired alternative (a1 to a3), (b1 to b3) or (c1 to c3) of step (i).

In a special embodiment of the present invention a solid composition comprising valsartan and at least one further active substance such as amlodipine and/or hydrochlorothiazide is obtained in form of a multilayer tablet such as a bilayer tablet or trilayer tablet when valsartan is combined with one additional active substance, or a bilayer or trilayer tablet when valsartan is combined with at least two additional active substances.

In another embodiment of the present invention the valsartan comprising tablet core can be compression coated with a granulate comprising at least one further active substance selected from calcium channel blockers such as amlodipine or its salts, and/or diuretic such as hydrochlorothiazide and/or indapamide, and/or holesterol lowering agent such as HMG-CoA reductase inhibitors, such as rosuvastatin, simvastatin, lovastatin, atorvastatin, fibrates, bile acid sequestrants and/or nicotinic acid. The compression coated solid composition according to present invention can be optionally film coated as described previously.

In still another embodiment of the present invention a solid composition comprising valsartan and at least one further active ingredient can be obtained by the coating -of valsartan comprising cores having a maximal diameter of round punches in the range of 500 µm to 12 mm or oval/capsule shaped punches in the range of lenght of 1 to 20 mm and width 1 to 15 mm with at least one coating comprising at least one active substance selected from calcium channel blockers such as amlodipine or its salts, and/or diuretic such as hydrochlorothiazide and/or indapamide, and /or holesterol lowering agent such as HMG-CoA reductase inhibitors, fibrates, bile acid sequestrants and/or nicotinic acid. Coating can be performed by state of the art coating processes and equipment such as pan coating or fluid bed coating where second active substance is applied onto the valsartan comprising cores in by suspension, solution, emulsion or powder coating.

### Components of the pharmaceutical composition

The solid pharmaceutical compositions of the present invention comprises valsartan or valsartan and amlodipine or their pharmaceutically acceptable salts, or valsartan and hydrochlorothiazide, or valsartan, amlodipine and hydrochlorothiazide and pharmaceutically acceptable excipients. The compositions for oral application can be in the form of a granulate, tablets, minitablets, powders, lozenges, sacchets, soft and hard gelatine capsules etc.

The compositions are preferably formulated in a unit dosage form, each dosage comprising about 1 to about 1000 mg, more usually about 40 to about 320 mg of valsartan. The term »unit dosage form« refers to physically discrete units suitable as unitary dosages for human subjects and other mammals, each unit comprising a predetermined quantity of valsartan calculated to produce the desired therapeutic effect, in association with a suitable pharmaceutical excipient, e.g. a tablets, or a capsule, the latter comprising minitablets or a granulate of a granulate in mixture with other non-granulated excipients. Valsartan can be present in different physical forms, e.g. in an amorphous form, in one or several crystalline form(s) (e.g. anhydrous, solvated or hydrated forms), in the form of a mixture of different crystal forms (e.g. anhydrous, solvated or hydrated forms) or as any mixture of an amorphous form and crystal form(s) (e.g. anhydrous, solvated or hydrated forms). Each of these forms is included in the term "valsartan" as used in the present invention.

Amlodipine or its pharmaceutically acceptable salts can be present in any solid physical form, e.g. in an amorphous form, in one or several crystal form(s) (e.g. anhydrous, solvated or hydrated forms), in the form of a mixture of different crystal forms (e.g. anhydrous, solvated or hydrated forms) or as any mixture of an amorphous form and crystal form(s) (e.g. anhydrous, solvated or hydrated forms). The composition are preferably formulated in a unit dosage for, each dosage comprising about 1 to about 20 mg, more usually about 5 to about 10 mg of amlodipine or its pharmaceutically acceptable salts.

The diuretic, preferably hydrochlorothiazide, can be present in any solid physical form, e.g. in an amorphous form, in one or several crystal form(s) (e.g. anhydrous, solvated or hydrated forms), in the form of any mixture of different crystal forms (e.g. anhydrous, solvated or hydrated forms) or as any mixture of an amorphous form and crystal form(s) (e.g. anhydrous, solvated or hydrated forms). The compositions are preferably formulated in a unit dosage for, each dosage comprising about 1 to about 25 mg, more usually about 12.5 to about 25 mg of hydrochlorothiazide.

The pharmaceutical excipients particularly include binders, disintegrants, diluents and lubricants. Other and further excipients can also be comprised.

### Binders

The composition according to the invention can also comprise binders, such as polyvinylpyrrolidone (povidone), copovidone, microcrystalline cellulose, hydroxyethylcellulose, hydroxypropylcellulose, hydroxypropylmethylcellulose or other cellulose ethers, starch, pregelatinised starch, or polymethacrylate, or mixtures thereof.

It is preferable to use a binder with good water solubility. In view of the practical insolubility of starch in cold water, there is a strong preference for binders with very good solubility in cold water. There is a variety of binders that have very good solubility in water, e.g. povidone of different K-values, i.e. low K-values such as 10 to 50. When microcrystalline cellulose is used as a binder, its content in the formulation has an influence on the processability of the product if a dry granulation process is used. Higher contents were generally favoured in the prior art for dry granulation processes. Surprisingly it was shown that microcrystalline cellulose which is a rather expensive excipient can partially or completely be substituted by lactose or other diluents, by using a defined particle size of valsartan and the technological process according to the present invention. It has been surprisingly found that in a wet aqueous granulation process the amount of microcrystalline cellulose can be minimized to less than 30% in weight in formulation. Lactose is also less expensive than microcrystalline cellulose and is from an economical viewpoint preferred over microcrystalline cellulose.
In a preferred embodiment of the invention the excipients include at least one binder selected from hydroxypropyl cellulose and povidone.

### Disintegrants

Further, the pharmaceutical compositions of the present invention may also comprise disintegrants, such as pregelatinsed starch, sodium starch glycolate, carboxymethylcellulose sodium (CMC-Na), cross-linked CMC-Na (croscarmellose sodium), polacrilin potassium, low-substituted hydroxypropylcellulose or mixtures thereof.

Crospovidone as a water-insoluble tablet disintegrant is also insoluble in most organic solvents and can be used in up to 5% concentration in direct compression, wet or dry granulation process. However, it is preferable to use a disintegrant that can be used in both wet and dry stages of the wet granulation process (intra- and extragranulary) so that the wicking and swelling ability of the disintegrant is best utilized. Crospovidone is very hygroscopic with a maximum moisture sorption of 60% (w/w) and is therefor not a disintegrant of first choice in the formulation of valsartan by an (aqueous) wet granulation process. Crospovidone could also cause the appearance of oxidation impurities of the oxygen sensitive active substances in the formulation.

As a potential disintegrant that can be used intra- and extragranulary, cross-linked carboxymethylcellulose sodium (croscarmellose sodium) is therefore preferable. Although it is insoluble in water, it rapidly swells to 4-8 time its original volume on contact with water.

It is particularly preferred in the present invention that the excipients include at least one disintegrant selected from starch and carboxymethylcellulose sodium, e.g. a cross-linked carboxymethylcellulose sodium (croscarmellose sodium).

### Diluents

The pharmaceutical compositions according to the invention can further comprise diluents such as microcrystalline cellulose, powdered cellulose, lactose (anhydrous or monohydrate), compressible sugars, fructose, dextranes, other sugars such as mannitol, sorbitol, xylitol, lactitol, isomalt, saccharose or a mixture thereof, dibasic anhydrous calcium phosphate (e.g. Di-Cafos A with a typical pH value of about 7-8 in a 10 % (w/w) suspension), calcium carbonate, calcium lactate, coprocessed multifunctional excipients such as, fructose and starch (commercially available as Advantose FS 95), microcrystalline cellulose and guar gum (commercially available as Avicel CE15), lactose and cellulose (commercially available as Cellactose and MicroceLac), F-Melt, calcium carbonate and sorbitol (commercially available as Formaxx), mannitol, superfine crospovidone (commercially available as Kollidon CL-SF) and polyvinyl acetate dispersion stabilized with povidone and sodium lauryl sulfate (commercially available as Kollicoat SR 30 D) (the multifunctional excipient is commercially available as Ludiflash), lactose, povidone (commercially available as Kollidon 30) and crospovidone (commercially available as Kollidon CL) (the multifunctional excipient is commercially available as Ludipress), microcrystalline cellulose, hypromellose and crospovidone (commercially available as PanExcea MCC333G), anhydrous lactose and lactitiol (commercially available as Pharmatose DCL40), microcrystalline cellulose and colloidal silicon dioxide (commercially available as Prosolv), corn starch and pregelatinized starch (commercially available as StarCap 1500), lactose and maize starch (commercially available as Star lac), xylitol and polydextrose (commercially available as Xylitab 100), xylitol and sodium carboxymethylcellulose (commercially available as Xylitab), sucrose and dextrin (commercially available as Di-Pac), microcrystalline cellulose and calcium phosphate (commercially available as Celocal), fructose and starch (commercially available as Advantose FS95), maltose (commercially available as Advantose 100), calcium carbonate and starch (commercially available as Barcroft CS90), Al₂(OH)₃, Mg₂(OH)₃ and sorbitol (commercially available as Barcroft Premix St), vinyl acetate and vinyl pyrrolidone (commercially available as Plasdone S-630 copovidone), calcium carbonate and acacia (commercially available as Carbofarma GA10), calcium carbonate and matodextrin (commercially available as Carbofarma GM11) or mixtures thereof. For the diluents used extragranularly it is important to select those having good compressibility and flowability such as those designed for direct compression and can be selected form coprocessed excipients wherein two or more excipients are combined into coprocessed single particles and/or processed single expients such as spray dryed or granulated excipient. It is preffered that extragranularly used excipients have average particle size measured by lased diffraction method in the range 30 to 500 µm, preferably 50 to 400 µm and even more preferably 70 to 300 µm.

By the means of the term "multifunctional excipient", different functions within the same excipent are meant, for example the same excipient could act as diluents, disintegrant and lubricant. However, individual functions could not be quantitatively evaluated.

Preferably, the excipients include at least one diluent selected from microcrystalline cellulose (however, this diluent should only be used in an amount of less than 30 weight %), lactose monohydrate, polyols and dibasic anhydrous calcium phosphate.

### Lubricants

The composition according to the invention may also comprise lubricants, such as stearic acid, metal salts of fatty acids with 12 to 24 carbon atoms such as magnesium stearate, calcium stearate, sodium lauryl sulphate, hydrogenated vegetable oil, hydrogenated castor oil, sodium stearyl fumarate, macrogols, or mixtures thereof.
It is preferred that the excipients include at least one lubricant, selected from stearic acid, magnesium stearate, calcium stearate, sodium stearyl fumarate and sodium lauryl sulphate.

### Further active ingredients

In a special embodiment of present invention valsartan can be combined with at least one further active ingredient selected from diuretics, antihypertensives, lipid regulators and antidiabetics in the form of free acid, free base, salt or ester. Antihypertensives can be selected but not limited to from following farmacological subgroups: angiotensin converting enzyme (ACE) inhibitors, angiotensin receptor blockers (ARBs), AT₁-receptor antagonists, calcium-channel blockers (CCBs) and antiadrenergics.

For example, ACE inhibitors may be selected from the group consisting of captopril, enalapril, lisinopril, trandolapril, cilazapril, ramipril, fosinopril, perindopril or a pharmaceutically acceptable salt thereof.

AT₁-receptor antagonist for use in the combined formulation may be selected from the group consisting of candesartan, irbesartan, losartan, olmesartan, telmisartan or pharmaceutically acceptable salts thereof.

The calcium channel blocker may be selected from the group consisting of amlodipine, diltiazem, felodipine, nifedipine, nitrendipine, nimodipine and verapamil and salts and/or esters thereof.

From the group of β-adrenergic blockers compounds like acebutol, atenolol, betaxolol, bisoprolol, metoprolol, and pharmaceutically acceptable salts can be selected. As mixed α-and β-adrenergic blockers, carvedilol may be included in the formulation.

Lipid regulators for combination with valsartan in the formulation of the present invention can be selected from of 3-hydroxy-3-methylglutaryl coenzyme A (HMG CoA) reductase inhibitors such as lovastatin, simvastatin, pravastatin, atorvastatin, fluvastatin, cerivastatin, rosuvastatin, and salts thereof.

The antidiabetic agent may be selected from the group of sulfonyl urea, meglitinides (such as nateglinide, repaglinide) and pharmaceutically acceptable salts thereof, thiazolidinediones (such as pioglitazone, rosiglitazone) and pharmaceutically acceptable salts thereof, alpha glucosidase inhibitors, incretin mimetics, or biguanides such as metformin or the like and the pharmaceutically acceptable salts thereof.

### Preferred quantities of ingredients

The preferred solid pharmaceutical composition of the present invention comprise (all percentages are weight-based, an optional coating is not included):
- 15-60%, preferably 20-60% of valsartan,
- 10-85%, preferably 15-85% of diluents,
- 1-20%, preferably 1-15% of disintegrant,
- 1-20%, preferably 1-15% of binder,
- 1-10%, preferably 1-8% of Ilubricant.

### Coatings

Optionally, cores/tablets can be coated with conventional materials used for film coating, i.e. as described in Pharmaceutical Coating Technology, 1995, edited by Graham Cole. Film coating formulations usually comprise the following components: polymer(s), plasticizer(s), colourant(s)/opacifier(s), vehicle(s). In film coating suspension also minor quantities of flavours, surfactants and waxes can be used. The majority of the polymers used in film coating are either cellulose derivatives, such as the cellulose ethers, or acrylic polymers and copolymers. Occasionally encountered are high molecular weight polyethylene glycols, polyvinyl pyrrolidone, polyvinyl alcohol and waxy materials.

In a special embodiment of the present invention cores/tablets can optionally be coated with a film coating having low permeability for gases such as oxygen and/or moisture from air. Such coatings are based on polymers which are preferably soluble in water, i.e. having solubility in water at 25°C of at least 1 g per 100 ml of water or at least one buffer solution with pH in the range 1 to 7. Typical polymers used for such coatings with a decreased permeability for gases are hydroxylpropyl cellulose, calcium or sodium carboxymethyl cellulose, graft copolymer of polyethylene glycol and polyvinyl alcohol (Kollicoat IR or Kollicoat Protect), polyvinyl alcohol (such as Opadry HP II or Opadry AMB), calcium carboxymethyl cellulose, aminoalkyl methacrylate copolymers (such as Eudragit EPO), methacrylic acid copolymers (such as Eudragit L types). Coating can further comprise other excipients selected from plasticizers, pigments and colorants, antitacking agents, surfactants, water soluble pore formers.

Typical cellulose ethers are hydroxyethylcellulose, hydroxypropylcellulose, hydroxypropylmethylcellulose, or methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups: polyols (glycerol, propylene glycol, macrogol(s)), organic esters (phthalate esters, dibutyl sebacetate, citrate esters, triacetin), oils/glycerides (castor oil, acetylated monoglycerides, fractionated coconut oil).

Colourants/pigments are classified into several groups: organic dyes and their lakes, inorganic colours, natural colours. Combination of different materials from each group can be combined in defined ratios. Film coating suspensions can be used as ready-to-make preparations which are available on the market.

Film coating dispersion can be prepared by using different solvents (water, alcohols, ketones, esters, chlorinated hydrocarbons), preferably water.

A composition of coating suspension (calculated on dry material) which comprises is particularly preferred:
- 1-99% by weight of polymer, preferably 1-95% of polymer,
- 1-50% by weight of plasticizer, preferably 1-40% of plasticizer,
- 0.1-20% of colourant/pigment, preferably 0.1-10% of colourant/pigment.

The pre-mixes as well as the pharmaceutical composition and the final solid oral dosage form may be embedded in a gaseous mixture, wherein the oxygen may be present at a concentration of between approximately 0.1 % to 16 % (v/v), preferably at a concentration of below 12 % (v/v), most preferably at a concentration of below 8 % (v/v). Preferably nitrogen or argon can be used as inert gas atmosphere in the packaging procedure, wherein nitrogen is especially preferred.

Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister or a bottle.Specially preferred is packaging material having decreased permeability for oxygen and/or water vapour (moisture). Decreased permeability for moisture means that the water permeability is below 0.5 g/(m²*day). Decreased gas permeability is meaning gas, especially oxygen, permeability of less than 0.1cm³*day. Low moisture permeable primary packaging materials such as polychloro-3-fluoroethylene homopolymer/PVC laminate can be used with the thickness in the range 270 µm to 360 µm. In case of Alu/Alu blisters a multilayer laminate with the total thickness in the range 100 µm to 155 µm and thickness of aluminium foil layer in the range 5 µm to 100 µm, preferably 7 µm to 60 µm, can be used. Optionally, dosage forms comprosing valsartan or its pharmaceutically acceptable salt can be packed into primary packaging with desiccant. Desiccant, containing material having high capacity for moisture sorption such as silica, molecular sieve, zeolite, can be placed inside the packaging unit together with valsartan comprising dosage units such as tablets or capsules and/or in the closure system or can be incorporated into the walls of the primary packaging unit.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of adsorbents. Suitable adsorbents may be selected from the group of commercially available adsorbents such as humidity-activated oxygen adsorbers, ultraviolet-radiation-activated adsorbers, radiation-activated adsorbers, microwave-radiation-activated adsorbers, adsorbers activated by a combination of activation processes or adsorbers without necessity of activation. Examples of commercially available adsorbers are Ageless™ (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

The following examples illustrate the invention and are not intended to restrict the scope of the invention in any way. In the following examples, the water content was determined as loss on drying in a Mettler Toledo halogen moisture analyzer at 85°C for 20 minutes and at 105°C for 5 minutes. The total water content was determined according to Ph. Eur. 2.5.12. Water: Semi-micro determination, with a sample weight 150 mg to 250 mg. The physical characteristics of granulates and compression mixtures are determined according to Ph.Eur. 6.6., monograph 2.9.36 Powder flow.

### Examples

### Comparative Example 1

The formulation was prepared by dry granulation, i.e. the process in the absence of water, as described in EP1096932 B1. The linear increase of the described composition Valsartan/Amlodipin 80 mg/5 mg was prepared.

| **Composition** | |
|---|---|
| **Compactate (dry granulate)** | |
| Valsartan | 160.00 mg |
| Amlodipine besylate | 13.88 mg* |
| Avicel PH102 (microcrystalline cellulose) | 204.12 mg |
| Polyplasdone XL (crospovidone) | 40.00 mg |
| Aerosil 200 | 1.50 mg |
| Magnesium stearate | 5.00 mg |

| **Outer (extragranular) phase** | |
|---|---|
| Aerosil 200 | 1.50 mg |
| Magnesium stearate | 4.00 mg |
| | |
| Hardness of the cores | 96-141 N |
| Disintegration time of the cores | 30-40 s |
| Dimensions of the punches | Oval, lenght 13 mm, width 8 mm |
| Main pressure at tabletting | 9.7 kN |

| | |
|---|---|
| * corresponding to 10.00 mg of amlodipine | |

### Comparative Example 2

4.00 kg of valsartan, 2.05 kg of microcrystalline cellulose (commercially available as Avicel PH101), 75 g of povidone and 62.5 g of croscarmellose sodium were homogenized in a high-shear mixer Collette Gral 75.

3.30 kg of purified water was sprayed into the mixture of valsartan and excipients as listed above. The granulate obtained was dried in a fluid-bed dryer Glatt WSG 5 in one step at inlet air temperature 48-55.6°C for 2 hours and 20 minutes. The loss on drying (LOD) was 2.28% (performed at 85°C for 20 minutes).

The results of physical parameters (sieve analysis, bulk volume, tapped volume, Hausner index, Carr index) of the granulate obtained above are presented in a table below. According to the scale of flowability (Ph.Eur. 6.6., monograph 2.9.36 Powder flow), the granulate exhibited passable flow.

| Sieve analysis (pass a sieve with sieve openings): | |
|---|---|
| 71 µm | 41.50% |
| 125 µm | 63.45% |
| 250 µm | 82.20% |
| 500 µm | 95.45% |
| 710 µm | 100.00% |
| 1250 µm | 100.00% |
| Bulk volume | 2.14 mL/g |
| Tapped volume | 1.66 mL/g |
| Hausner index | 1.29 |
| Carr index | 22.4 |

1.50 kg of lactose monohydrate (commercially available as Tablettose 70), 37.5 g fo croscarmellose sodium, 50 g of colloidal anhydrous silica and 225 g of magnesium stearate were added to the granulate above and mixed in a biconic mixer. The LOD of the compression mixture was 2.04% (performed at 85°C for 20 minutes) and 1.98% (performed at 105°C for 5 minutes).

The results of physical parameters (sieve analysis, bulk volume, tapped volume, Hausner index, Carr index) of the compression mixture obtained above are presented in the table below. According to the scale of flowability (Ph.Eur. 6.6., monograph 2.9.36 Powder flow), the compression mixture exhibited passable flow.

| Sieve analysis (pass a sieve with a sieve openings): | |
|---|---|
| 71 µm | 35.65% |
| 125 µm | 59.50% |
| 250 µm | 86.10% |
| 500 µm | 96.90% |
| 710 µm | 100.00% |
| 1250 µm | 100.00% |
| Bulk volume | 2.12 mL/g |
| Tapped volume | 1.62 mL/g |
| Hausner index | 1.31 |
| Carr index | 23.6 |

The compression mixture was compressed into cores with a theoretical weight of 640.00 mg. Oval punches used have lenght 16.8 mm and width 8.5 mm with a score. Hardness of the cores was 111-155 N (average hardness of 20 cores was 134 N) at main pressure of tabletting 7.5 kN and the disintegration time was 60 seconds.

Cores were coated in an automatic coating pan with a water-based coating supension, comprising (calculated per dry material) hypromellose (70 w/w %), titanium dioxide (15 w/w %), E172 yellow (6.25 w/w %), E172 red (1.25 w/w %) and macrogol 4000 (7.5 w/w %). The theoretical weight of the film-coated tablets was 656.00 mg.

### Example 1

04.00 kg of valsartan, 2.05 kg of microcrystalline cellulose (commercially available as Avicel PH101), 75 g of povidone and 119 g of croscarmellose sodium were homogenized in a high-shear mixer Collette Gral 75.

3.30 kg of purified water was sprayed into the mixture of valsartan and excipients as listed above. The granulate obtained was dried in a fluid-bed dryer Glatt WSG 5 in two steps.
Step 1 was performed at an absolute humidity of the inlet air of 0.82-1.19 g/kg and inlet air temperature 25°C (set-point) for 5 hours. The loss on drying (LOD) was performed at 105°C for 5 minutes and at 85°C for 20 minutes. The wetted granulate was analysed after addition of purified water (initial (t=0) - before beginning of drying) through the whole process of drying of step 1.

| **Sample** | **LOD 85°C, 20 min** | **LOD 105°C, 5 min** | **Inlet air temperature** | **Temperature of product** | **Abs. humidity of of inlet air** |
|---|---|---|---|---|---|
| Initial (t=0) | / | 12.62% | 31°C | 11.8°C | 0.82 g/kg |
| t= 2 h | / | 5.80% | 28°C | 17.6°C | 1.01 g/kg |
| t=3 h | 2.04% | 1.83% | 27°C | 22.1°C | 1.18 g/kg |
| t=4h | 1.98% | / | 26°C | 26.3°C | 1.07 g/kg |
| t=5 h | / | 1.30% | 26°C | 26.4°C | 1.19 g/kg |

| | | | | | |
|---|---|---|---|---|---|
| / not measured | | | | | |

After 5 h, the step 2 drying was followed subsequently to step 1 at absolute humidity of the inlet air 1.13-1.20 g/kg and inlet air temperature 55°C (set-point) for 18 minutes.

| **Sample** | **LOD 105°C, 5 min** | **Inlet air temperature** | **Temperature of product** | **Absolute humidity of of inlet air** |
|---|---|---|---|---|
| t=11 min | 1.18% | 37.5°C | 30°C | 1.13 g/kg |
| t=18 min | 1.18% | 43.9°C | 33°C | 1.20 g/kg |

347 g of amlodipine besylate, 1.13 kg of lactose monohydrate (commercially available as Tablettose 70), 50 g of colloidal anhydrous silica and 225 g of magnesium stearate were added to the granulate above and mixed in a biconic mixer. The LOD of the compression mixture was 1.79% (performed at 85°C for 20 minutes) and 1.74% (performed at 105°C for 5 minutes). The compression mixture was compressed into cores with a theoretical weight of 320.00 mg. Oval punches used have lenght 13 mm and width 8 mm. Hardness of the cores was 96-110 N (average hardness of 20 cores was 103 N) at main pressure of tabletting 6.9 kN and disintegration time was 30 seconds.
Cores were coated in an automatic coating pan with a ready-to-make mixture Opadry II white with addition of yellow iron oxide, both suspended in purified water. The theoretical weight of the film-coated tablets was 328.00 mg.

### Example 2

4.00 kg of valsartan, 2.05 kg of microcrystalline cellulose (commercially available as Avicel PH200), 75 g of povidone and 119 g of croscarmellose sodium were homogenized in a high-shear mixer Collette Gral 75.
3.30 kg of purified water was sprayed into the mixture of valsartan and excipients as listed above. The granulate obtained was dried in a fluid-bed dryer Glatt WSG 5 in two steps.
The step 1 was performed at absolute humidity of the inlet air 0.71-0.97 g/kg and inlet air temperature 25°C (set-point) for 5 hours. The LOD was performed at 105°C for 5 minutes and at 85°C for 20 minutes. The wetted granulate was analysed after addition of purified water (initial (t=0) - before beginning of drying) through the whole process of drying of step 1.

| **Sample** | **LOD 85°C, 20 min** | **LOD 105°C, 5 min** | **Inlet air temperature** | **Temperature of product** | **Abs.humidity of to inlet air** |
|---|---|---|---|---|---|
| Initial (t=0) | 28.7% | 15.28% | 29.6°C | / | 0.90 g/kg |
| t=1 h | 20.08% | 9.65% | 29.2°C | 12.1°C | 0.71 g/kg |
| t= 2 h | 12.07% | 9.26% | 27.5°C | 11.8°C | 0.78 g/kg |
| t=3 h | 2.98% | 2.91% | 27.1°C | 21.9°C | 0.85 g/kg |
| t=4h | 1.54% | 1.30% | 28.2°C | 26.0°C | 0.75 g/kg |
| t=5 h | / | 1.26% | 27.8°C | 27.5°C | 0.97 g/kg |

| | | | | | |
|---|---|---|---|---|---|
| / not measured | | | | | |

After 5 h, the step 2 drying was followed subsequently to step 1 at absolute humidity of the inlet air 0.83-0.89 g/kg and inlet air temperature 55°C (set-point) for 8 minutes.

| **Sample** | **LOD 85°C, 20 min** | **LOD 105°C, 5 min** | **Inlet air temperature** | **Temperature of product** | **Abs. Humidity of of inlet air** |
|---|---|---|---|---|---|
| t=4 min | / | 1.23% | 33.9°C | 30°C | 0.89 g/kg |
| t=8 min | 1.18% | 1.16% | 43.1°C | 35°C | 0.83 g/kg |

| | | | | | |
|---|---|---|---|---|---|
| / not measured | | | | | |

The results of physical parameters (sieve analysis, bulk volume, tapped volume, Hausner index, Carr index) of the granulate obtained above are presented in a table below. According to the scale of flowability (Ph.Eur. 6.6., monograph 2.9.36 Powder flow), the granulate exhibited fair flow.

| Sieve analysis (pass a sieve with sieve openings): | |
|---|---|
| 71 µm | 37.00% |
| 125 µm | 67.50% |
| 250 µm | 93.00% |
| 500 µm | 100.00% |
| 710 µm | 100.00% |
| 1250 µm | 100.00% |
| Flowability time | 70 seconds |
| Bulk volume | 2.40 mL/g |
| Tapped volume | 1.92 mL/g |
| Hausner index | 1.25 |
| Carr index | 20.0 |

347 g of amlodipine besylate, 1.13 kg of lactose monohydrate (commercially available as Tablettose 70), 50 g of colloidal anhydrous silica and 225 g of magnesium stearate were added to the granulate above and mixed in a biconic mixer. The LOD of the compression mixture was 1.34% (performed at 85°C for 20 minutes) and 1.28% (performed at 105°C for 5 minutes).

The results of physical parameters (sieve analysis, bulk volume, tapped volume, Hausner index, Carr index) of the compression mixture obtained above are presented in a table below. According to the scale of flowability (Ph.Eur. 6.6., monograph 2.9.36 Powder flow), the compression mixture exhibited fair flow.

| Sieve analysis (pass a sieve with sieve openings): | |
|---|---|
| 71 µm | 33.0% |
| 125 µm | 58.5% |
| 250 µm | 91.5% |
| 500 µm | 100.00% |
| 710 µm | 100.00% |
| 1250 µm | 100.00% |
| Flowability time | 44.1 seconds |
| Bulk volume | 2.30 mL/g |
| Tapped volume | 1.86 mL/g |
| Hausner index | 1.24 |
| Carr index | 19.1 |

The compression mixture was compressed into cores with a theoretical weight of 320.00 mg. Oval punches used have lenght 13 mm and width 8 mm. Hardness of the cores was 89-110 N at main pressure of tabletting 6.5 kN and disintegration time was 30-35 seconds.
Cores were coated as described in Example 1.

### Example 3

4.00 kg of valsartan, 2.05 kg of microcrystalline cellulose (commercially available as Avicel PH200), 75 g of povidone and 119 g of croscarmellose sodium were homogenized in a high-shear mixer Collette Gral 75.

4.00 kg of purified water was sprayed into the mixture of valsartan and excipients as listed above. The granulate obtained was dried in a fluid-bed dryer Glatt WSG 5 in 2 steps.

The step 1 was performed at absolute humidity of the inlet air 0.76-1.12 g/kg and inlet air temperature 25°C (set-point) for 5.5 hours. The LOD was performed at 105°C for 5 minutes and at 85°C for 20 minutes. The wetted granulate was analysed after addition of purified water (initial (t=0) - before beginning of drying) through the whole process of drying of step 1.

| **Sample** | **LOD 85°C, 20 min** | **LOD 105°C, 5 min** | **Inlet air temperature** | **Temperature of product** | **Abs. humidity of inlet air** |
|---|---|---|---|---|---|
| Initial (t=0) | 32.11% | 12.85% | 31.8°C | 11.6°C | 0.76 g/kg |
| t=1 h | 20.89% | 11.34% | 27.9°C | 11.4°C | 0.85 g/kg |
| t= 2 h | 12.88% | 10.28% | 28.0°C | 11.8°C | 1.06 g/kg |
| t=3 h | 5.15% | 4.98% | 27.8°C | 15.2°C | 1.00 g/kg |
| t=4h | 1.91% | 1.63% | 27.7°C | 25.8°C | 1.08 g/kg |
| t=5 h | 1.71% | 1.35% | 28.2°C | 27.2°C | 1.12 g/kg |
| t=5.5 h | 1.48% | 1.25% | 28.3°C | 27.1°C | 1.10 g/kg |

After 5.5 h, the step 2 drying was followed subsequently to step 1 at absolute humidity of the inlet air 1.10-1.12 g/kg and inlet air temperature 55°C (set-point) for 14 minutes.

| **Sample** | **LOD 85°C, 20 min** | **LOD 105°C, 5 min** | **Inlet air temperature** | **Temperature of product** | **Abs. humidity of of inlet air** |
|---|---|---|---|---|---|
| t=8 min | / | 1.25% | 36.7°C | 30°C | 1.10 g/kg |
| t=14 min | / | 1.12% | 42.8°C | 33°C | 1.12 g/kg |

| | | | | | |
|---|---|---|---|---|---|
| / not measured | | | | | |

347 g of amlodipine besylate, 1.13 kg of lactose monohydrate (commercially available as Tablettose 70), 50 g of colloidal anhydrous silica and 225 g of magnesium stearate were added to the granulate above and mixed in a biconic mixer. The LOD of the compression mixture was 1.28% (performed at 85°C for 20 minutes) and 1.12% (performed at 105°C for 5 minutes). The compression mixture was compressed into cores with a theoretical weight of 320.00 mg. Oval punches used have lenght 13 mm and width 8 mm. Hardness of the cores was 75-98 N at main pressure of tabletting 6.2 kN and disintegration time was 30 seconds.
Cores were coated as described in Example 1

### Example 4

4.00 kg of valsartan, 2.05 kg of microcrystalline cellulose (commercially available as Avicel PH101), 75 g of povidone and 62.5 g of croscarmellose sodium were homogenized in a high-shear mixer Collette Gral 75.

3.00 kg of purified water was sprayed into the mixture of valsartan and excipients as listed above. The granulate obtained was dried in a fluid-bed dryer WSG 5 in 2 steps.
The step 1 was performed at absolute humidity of the inlet air 0.70-1.56 g/kg and inlet air temperature 25°C (set-point) for 6 hours. The LOD of the granulate was performed at 105°C for 5 minutes and at 85°C for 20 minutes in comparison to analysis of total water content, performed by Karl-Fischer titatration. The wetted granulate was analysed after addition of purified water (initial (t=0) - before beginning of drying) through the whole process of drying of step 1.

| **Sample** | **Total water content** | **LOD 85°C, 20 min** | **LOD 105°C, 5 min** | **Inlet air temperature** | **Abs. humidity of inlet air** |
|---|---|---|---|---|---|
| Initial (t=0) | 32.27% | 27.27% | 13.29% | / | / |
| t=1 h | 21.30% | 17.80% | 8.78% | 27.0°C | 0.68 g/kg |
| t= 2 h | 12.20% | 11.65% | 5.57% | 25.7°C | 0.84 g/kg |
| t=3 h | 6.05% | 5.72% | 4.32% | 25.8°C | 0.94 g/kg |
| t=4h | 2.58% | 2.25% | 1.70% | 25.5°C | 0.90 g/kg |
| t=5 h | 1.92% | 1.73% | 1.25% | 25.8°C | 1.10 g/kg |
| t=6 h | 1.68% | 1.57% | 1.16% | 26.0°C | 1.56 g/kg |

| | | | | | |
|---|---|---|---|---|---|
| / not perfomed | | | | | |

After 6 h, the step 2 drying followed subsequently to step 1 at absolute humidity of the inlet air 1.43-1.50 g/kg and inlet air temperature 35°C (set-point) for 21 minutes.

| **Sample** | **Total water content** | **LOD 85°C, 20 min** | **LOD 105°C, 5 min** | **Inlet air temp.** | **Temp. of product** | **Abs. humidity of inlet air** |
|---|---|---|---|---|---|---|
| t=11 min | 1.76% | 1.55% | 1.31% | 35°C | 30°C | 1.43 g/kg |
| t=18 min | 1.69% | 1.26% | 1.25% | 40°C | 35°C | 1.50 g/kg |

1.50 kg of lactose monohydrate (commercially available as Tablettose 70), 37.5 g of croscarmellose sodium, 50 g of colloidal anhydrous silica and 225 g of magnesium stearate were added to the granulate above and mixed in a high-shear mixer. The LOD of the compression mixture was 1.31% (performed at 85°C for 20 minutes) and 1.26% (performed at 105°C for 5 minutes). The compression mixture was compressed into cores with a theoretical weight of 640.00 mg. Oval punches used have lenght 16 mm and width 8.5 mm with a score. Hardness of the cores was 88-105 N at main pressure of tabletting 5.0 kN and disintegration time was 25 seconds.

Cores were coated in an automatic coating pan with a water-based coating supension, comprising (calculated per dry material) hypromellose (70 w/w %), titanium dioxide (15 w/w %), E172 yellow (6.25 w/w %), E172 red (1.25 w/w %) and macrogol 4000 (7.5 w/w %). The theoretical weight of the film-coated tablets was 656.00 mg.

### Example 5

4.00 kg of valsartan, 2.05 kg of microcrystalline cellulose (commercially available as Avicel PH101), 75 g of povidone and 62.5 g of croscarmellose sodium were homogenized in a high-shear mixer Collette Gral 75.
3.00 kg of purified water was sprayed into the mixture of valsartan and excipients as listed above. The granulate obtained was dried in a fluid-bed dryer Glatt WSG 5 in 2 steps.
The step 1 was performed at absolute humidity of the inlet air 0.69-0.93 g/kg and inlet air temperature 25°C (set-point) for 6 hours. The LOD of the granulate was performed at 105°C for 5 minutes and at 85°C for 20 minutes in comparison to analysis of total water content, performed by Karl-Fischer titration. The homogenised mixture of valsartan and excipients was analysed after homogenisation prior to granulation (homogenised mixture). The wetted granulate was analysed after addition of purified water (initial (t=0) - before beginning of drying) through the whole process of drying of step 1.

| **Sample** | **Total water content** | **LOD 85°C, 20 min** | **LOD 105°C, 5 min** | **Inlet air temp.** | **Abs. humidity of inlet air** |
|---|---|---|---|---|---|
| Homogenised mixture | 2.49% | 2.07% | 1.87% | / | / |
| Initial (t=0) | 32.84% | 31.89% | 19.18% | 28.3°C | 0.93 g/kg |
| t=1 h | 20.89% | 19.31% | 13.39% | 27.3°C | 0.72 g/kg |
| t= 2 h | 11.42% | 11.15% | 10.19% | 26.6°C | 0.69 g/kg |
| t=3 h | 4.09% | 3.86% | 3.43% | 26.2°C | 0.84 g/kg |
| t=4h | 2.23% | 2.14% | 1.70% | 26.0°C | 0.85 g/kg |
| t=5 h | 1.86% | 1.43% | 1.59% | 26.4°C | 0.75 g/kg |
| t=6 h | 1.53% | 1.38% | 0.95% | 26.5°C | 0.75 g/kg |

| | | | | | |
|---|---|---|---|---|---|
| / not performed | | | | | |

After 6 h, the step 2 drying was followed subsequently to step 1 at absolute humidity of the inlet air 0.74-0.75 g/kg and inlet air temperature 55°C (set-point) for 16 minutes.

| **Sample** | **Total water content** | **LOD 85°C, 20 min** | **LOD 105°C, 5 min** | **Inlet air temp.** | **Temp. of product** | **Abs. Humidity of of inlet air** |
|---|---|---|---|---|---|---|
| t=11 min | 1.46% | 1.26% | 1.27% | 36.8°C | 30°C | 0.75 g/kg |
| t=16 min | 1.34% | 1.20% | 1.27% | 42.0°C | 35°C | 0.74 g/kg |

1.50 kg of lactose monohydrate (commercially available as Tablettose 70), 37.5 g of croscarmellose sodium, 50 g of colloidal anhydrous silica and 225 g of magnesium stearate were added to the granulate above and mixed in a high-shear mixer. The LOD of the compression mixture was 1.29% (performed at 85°C for 20 minutes) and 1.20% (performed at 105°C for 5 minutes).The compression mixture was compressed into cores with a theoretical weight of 640.00 mg. Oval punches used have lenght 16 mm and width 8.5 mm with a score. Hardness of the cores was 85-106 N at main pressure of tabletting 5.4 kN and disintegration time was 30 seconds.

Cores were coated as described in Example 4.

### Examples 6a-d

The composition of the valsartan granulate and the process of preparation and drying of the granulate was the same as described above in Example 1. The use of different extragranular diluents was used: dibasic anhydous calcium phosphate, mannitol, xylitol and isomalt for Examples 6a-d, subsequently.

| **Composition** | **Example 6a** | **Example 6b** | **Example 6c** | **Example 6d** |
|---|---|---|---|---|
| **Granulate** | | | | |
| Valsartan | 160.00 mg | 160.00 mg | 160.00 mg | 160.00 mg |
| Microcrystalline cellulose | 82.00 mg | 82.00 mg | 82.00 mg | 82.00 mg |
| Povidone | 3.00 mg | 3.00 mg | 3.00 mg | 3.00 mg |
| Croscarmellose sodium | 4.75 mg | 4.75 mg | 4.75 mg | 4.75 mg |
| Purified water* | q.s. | q.s. | q.s. | q.s. |

| **Extragranular phase** | | | | |
|---|---|---|---|---|
| Amlodipine besylate | 13.88 mg | 13.88 mg | 13.88 mg | 13.88 mg |
| Dibasic anhydrous calcium phosphate¹⁾ | 45.37 mg | / | / | / |
| Mannitol²⁾ | / | 45.37 mg | / | / |
| Xylitol³⁾ | / | / | 45.37 mg | / |
| Isomalt⁴⁾ | / | / | / | 45.37 mg |
| Colloidal anhydro-silica | 2.00 mg | 2.00 mg | 2.00 mg | 2.00 mg |
| Magnesium stearate | 9.00 mg | 9.00 mg | 9.00 mg | 9.00 mg |
| | | | | |
| Hardness of the cores | 81-108 N | 93-111 N | 86-108 N | 81-108 N |
| Disintegration time of the cores | 23 s | 35-60 s | 40-45 s | 2.5-4 min |
| Dimensions of the punches | Oval, lenght 13 mm, width 8 mm | Oval, lenght 13 mm, width 8 mm | Oval, lenght 13 mm, width 8 mm | Oval, lenght 13 mm, width 8 mm |
| Main pressure at tabletting | 6.4 kN | 5.9 kN | 6.6 kN | 5.9 kN |

| | | | | |
|---|---|---|---|---|
| * not present in the final product, evaporates during drying / not included ¹⁾ commercially available as Di-Cafos A ²⁾ commercially available as Partec M200 ^{.3)} commercially available as Xylitab ⁴⁾ commercially available as GalenlQ | | | | |

Cores are optionally coated as described in Examples 1 or 4.

### Example 7

The composition of the granulate and the process of preparation of the granulate was the same as in Example 6a with the dividing of the croscarmellose sodium between granulate and extragranular phase.

| **Composition** | **Example 6a** |
|---|---|
| **Granulate** | |
| Valsartan | 160.00 mg |
| Microcrystalline cellulose | 82.00 mg |
| Povidone | 3.00 mg |
| Croscarmellose sodium | 3.10 mg |
| Purified water* | q.s. |

| **Extragranular phase** | |
|---|---|
| Amlodipine besylate | 13.88 mg |
| Dibasic anhydrous calcium phosphate¹⁾ | 45.37 mg |
| Croscarmellose sodium | 1.65 mg |
| Colloidal anhydrous silica | 2.00 mg |
| Magnesium stearate | 9.00 mg |

| | |
|---|---|
| * not present in the final product, evaporates during drying ¹⁾ commercially available as Di-Cafos A | |

Cores are optionally coated as described in Examples 1 or 4.

### Examples 8a-b

The composition of the valsartan granulate and the process of preparation and drying of the granulate was the same as described above in Example 1. Furthermore, the colloidal anhydrous silica was excluded from the composition of Example 6a in Example 8a. In Example 8b, the amount of magnesium stearate has been cutted to the half in comparison to Example 8a.

| **Composition** | **Example 8a** | **Example 8b** |
|---|---|---|
| **Granulate** | | |
| Valsartan | 160.00 mg | 160.00 mg |
| Microcrystalline cellulose | 82.00 mg | 82.00 mg |
| Povidone | 3.00 mg | 3.00 mg |
| Croscarmellose sodium | 4.75 mg | 4.75 mg |
| Purified water* | q.s. | q.s. |

| **Extragranular phase** | | |
|---|---|---|
| Amlodipine besylate | 13.88 mg | 13.88 mg |
| Dibasic anhydrous calcium phosphate¹⁾ | 47.37 mg | 51.87 mg |
| Magnesium stearate | 9.00 mg | 4.50 mg |
| | | |
| Hardness of the cores | 89-105 N | 92-116 N |
| Disintegration time of the cores | 35 s | 30 s |
| Dimensions of the punches | Oval, lenght 13 mm, width 8 mm | Oval, lenght 13mm, width 8 mm |
| Main pressure at tabletting | 6.9 kN | 6.2 kN |

| | | |
|---|---|---|
| * not present in the final product, evaporates during drying ¹⁾ commercially available as Di-Cafos A | | |

Cores are optionally coated as described in Examples 1 or 4.

### Examples 9a-e

### Preparation of the valsartan layer

4.00 kg of valsartan, 2.05 kg of microcrystalline cellulose (commercially available as Avicel PH200), 75 g of povidone and 62.5 g of croscarmellose sodium were homogenized in a high-shear mixer Collette Gral 75. 3.00 kg of purified water was sprayed into the mixture of valsartan and excipients as listed above. The granulate obtained was dried in a fluid-bed dryer Glatt WSG 5 in 2 steps.
The step 1 was performed at absolute humidity of the inlet air 0.89-1.53 g/kg and inlet air temperature 25°C (set-point) for 6 hours.

After 6 h, the step 2 drying was followed subsequently to step 1 at absolute humidity of the inlet air 1.28 g/kg and inlet air temperature 55°C (set-point) for 12 minutes. The LOD of the granulate was performed at 105°C for 5 minutes and at 85°C for 20 minutes and the results were 1.35% and 1.29%, subsequently.

1.50 kg of lactose monohydrate (commercially available as Tablettose 70), 37.5 g of croscarmellose sodium, 50 g of colloidal anhydrous silica and 225 g of magnesium stearate were added to the granulate above and mixed in a high-shear mixer. The LOD of the compression mixture was 1.26% (performed at 85°C for 20 minutes) and 1.23% (performed at 105°C for 5 minutes). The theoretical weight of the valsartan layer was 320.00 mg.

**Composition of the valsartan layer**

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 2.50 mg |
| Purified water* | q.s. |

| **Extragranular phase** | |
|---|---|
| Lactose monohydrate | 60.00 mg |
| Croscarmellose sodium | 1.50 mg |
| Colloidal anhydrous silica | 2.00 mg |
| Magnesium stearate | 9.00 mg |

| | |
|---|---|
| * not present in the final product, evaporates during drying | |

### Preparation of the amlodipine layer

347 g of amlodipine besylate, 6.64 kg of lactose monohydrate, dibasic anhydrous calcium phosphate, mannitol, xylitol and isomalt for Examples 9a-e, subsequently, 1.35 kg of pregelatinised starch, 0.55 kg of sodium starch glycolate, 27.5 g of colloidal anhydrous silica and 90 g of magnesium stearate were homogeneously mixed in a biconic mixer. The theoretical weight of the amlodipine layer was 360 mg.

**Composition of the amlodipine layer**

| **Composition** | **Example 9a** | **Example 9b** | **Example 9c** | **Example 9d** | **Example 9e** |
|---|---|---|---|---|---|
| Amlodipine besylate | 13.88 mg | 13.88 mg | 13.88 mg | 13.88 mg | 13.88 mg |
| Lactose monohydrate | 265.42 mg | | | | |
| Dibasic anhydrous calcium phosphate²⁾ | / | 265.42 mg | / | / | / |
| Mannitol³⁾ | / | / | 265.42 mg | / | / |
| Xylitol⁴⁾ | / | / | / | 265.42 mg | / |
| Isomalt⁵⁾ | / | / | / | / | 265.42 mg |
| Pregelatinised starch | 54.00 mg | 54.00 mg | 54.00 mg | 54.00 mg | 54.00 mg |
| Sodium starch glycolate | 22.00 mg | 22.00 mg | 22.00 mg | 22.00 mg | 22.00 mg |
| Colloidal anhydrous silica | 1.10 mg | 1.10 mg | 1.10 mg | 1.10 mg | 1.10 mg |
| Magnesium stearate | 3.60 mg | 3.60 mg | 3.60 mg | 3.60 mg | 3.60 mg |

| | | | | | |
|---|---|---|---|---|---|
| / not included ¹⁾ commercially available as Tablettose 70 ²⁾ commercially available as Di-Cafos A ³⁾ commercially available as Partec M200 ⁴⁾ commercially available as Xylitab ⁵⁾ commercially available as GalenlQ | | | | | |

After preparation of both valsartan and amlodipine layers, they are compressed into bilayer tablets of Examples 9a-e using automatic bilayer rotary tabletting machine. The theoretical weight of the bilayer tablet is 680.00 mg.
The bilayer tablets are optionally coated, using coating layer as described in Examples 1 or 4. The theoretical weight of coated bilayer tablet is 700.00 mg.

### Example 10

The composition of the valsartan granulate and the process of preparation and drying of the granulate for the valsartan layer was the same as described above in Example 1. The amlodipine layer was prepared as direct compression mixture.

**Composition of the valsartan layer**

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 4.00 mg |
| Purified water* | q.s. |

| **Extragranular phase** | |
|---|---|
| Lactose monohydrate | 60.00 mg |
| Colloidal anhydrous silica | 2.00 mg |
| Magnesium stearate | 9.00 mg |

| | |
|---|---|
| * not present in the final product, evaporates during drying | |

**Composition of the amlodipine layer**

| **Composition** | |
|---|---|
| Amlodipine besylate | 13.88 mg |
| Dibasic anhydrous calcium phosphate¹⁾ | 267.12 mg |
| Croscarmelose sodium | 4.30 mg |
| Colloidal anhydrous silica | 1.10 mg |
| Magnesium stearate | 3.60 mg |

| | |
|---|---|
| ¹⁾ commercially available as Di-Cafos A | |

The theoretical weight of the valsartan layer was 320.00 mg and of the amlodipine layer 290.00 mg. The total weight of the final bilayer tablet was 610.00 mg. The bilayer tablets are optionally coated, using coating layer as described in Examples 1 or 4.

### Examples 11a-e

The composition of the valsartan granulate and the process of preparation and drying of the granulate for the valsartan layer was the same as described above in Example 1. The use of different diluents was used: lactose monohydrate, dibasic anhydrous calcium phosphate, mannitol, xylitol and isomalt for Examples 11 a-e, subsequently.

| **Composition** | **Example 11a** | **Example 11b** | **Example 11c** | **Example 11d** | **Example 11e** |
|---|---|---|---|---|---|
| **Granulate** | | | | | |
| Valsartan | 160.00 mg | 160.00 mg | 160.00 mg | 160.00 mg | 160.00 mg |
| Microcrystalline cellulose | 82.00 mg | 82.00 mg | 82.00 mg | 82.00 mg | 82.00 mg |
| Povidone | 3.00 mg | 3.00 mg | 3.00 mg | 3.00 mg | 3.00 mg |
| Croscarmellose sodium | 4.75 mg | 4.75 mg | 4.75 mg | 4.75 mg | 4.75 mg |
| Purified water* | q.s. | q.s. | q.s. | q.s. | q.s. |

| **Extragranular phase** | | | | | |
|---|---|---|---|---|---|
| Amlodipine besylate | 13.88 mg | 13.88 mg | 13.88 mg | 13.88 mg | 13.88 mg |
| Hydrochlorothiazide | 12.50 mg | 12.50 mg | 12.50 mg | 12.50 mg | 12.50 mg |
| Lactose monohydrate¹ | 32.87 mg | / | / | / | / |
| Dibasic anhydrous calcium phosphate²⁾ | / | 32.87 mg | / | / | / |
| Mannitol³⁾ | / | / | 32.87 mg | / | / |
| Xylitol⁴⁾ | / | / | / | 32.87 mg | / |
| Isomalt⁵⁾ | / | / | / | / | 32.87 mg |
| Colloidal anhydrous silica | 2.00 mg | 2.00 mg | 2.00 mg | 2.00 mg | 2.00 mg |
| Magnesium stearate | 9.00 mg | 9.00 mg | 9.00 mg | 9.00 mg | 9.00 mg |

| | | | | | |
|---|---|---|---|---|---|
| * not present in the final product, evaporates during drying / not included ¹⁾ commercially available as Tablettose 70 ²⁾ commercially available as Di-Cafos A ³⁾ commercially available as Partec M200 ⁴⁾ commercially available as Xylitab ⁵⁾ commercially available as GalenlQ | | | | | |

### Example 12

The composition of the valsartan granulate and the process of preparation and drying of the granulate for the valsartan layer was the same as described above in Example 1. The extragranular additive was dibasic anhydous calcium phosphate (commercially available as Di-Cafos A).

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 5.90 mg |
| Purified water* | q.s. |

| **Extragranular phase** | |
|---|---|
| Amlodipine besylate | 13.88 mg |
| Hydrochlorothiazide | 12.50 mg |
| Dibasic anhydrous calcium phosphate¹⁾ | 113.72 mg |
| Magnesium stearate | 9.00 mg |

| | |
|---|---|
| * not present in the final product, evaporates during drying ¹⁾ commercially available as Di-Cafos A | |

### Example 13

The composition of the valsartan granulate and the process of preparation and drying of the granulate was the same as described above in Example 1. The disintegrant croscarmellose sodium is divided between the granulate and extragranular phase.

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 3.90 mg |
| Purified water* | q.s. |

| **Extragranular phase** | |
|---|---|
| Amlodipine besylate | 13.88 mg |
| Hydrochlorothiazide | 12.50 mg |
| Croscarmellose sodium | 2.00 mg |
| Dibasic anhydrous calcium phosphate¹⁾ | 113.72 mg |
| Magnesium stearate | 9.00 mg |

| | |
|---|---|
| * not present in the final product, evaporates during drying ¹⁾ commercially available as Di-Cafos A | |

### Example 14

The composition of the valsartan granulate and the process of preparation and drying of the granulate for the valsartan layer was the same as described above in Example 1. The amlodipine and hydrochlorothiazide layer was prepared as direct compression mixture.

**Composition of the valsartan layer**

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 4.00 mg |
| Purified water* | q.s. |

| **Extragranular phase** | |
|---|---|
| Lactose monohydrate | 60.00 mg |
| Colloidal anhydrous silica | 2.00 mg |
| Magnesium stearate | 9.00 mg |

| | |
|---|---|
| * not present in the final product, evaporates during drying | |

**Composition of the amlodipine and hydrochlorothiazide layer**

| **Composition** | |
|---|---|
| Amlodipine besylate | 13.88 mg |
| Hydrochlorothiazide | 12.50 mg |
| Dibasic anhydrous calcium phosphate¹⁾ | 254.62 mg |
| Croscarmelose sodium | 4.30 mg |
| Colloidal anhydrous silica | 1.10 mg |
| Magnesium stearate | 3.60 mg |

| | |
|---|---|
| ¹⁾ commercially available as Di-Cafos A | |

### Examples 15a-b

The composition of the valsartan granulate and the process of preparation and drying of the granulate for the valsartan layer was the same as described above in Example 1. The amlodipine layer for the strenghts 5 mg and 10 mg of amlodipine in Examples 15a and 15b, subsequently, was prepared as direct compression mixture. In both Examples 15a and 15b, the valsartan layer is the same as described in the table below.

**Composition of the valsartan layer**

| **Composition** | |
|---|---|
| **Granulate** | |
| Valsartan | 320.00 mg |
| Microcrystalline cellulose | 164.00 mg |
| Povidone | 6.00 mg |
| Croscarmellose sodium | 8.00 mg |
| Purified water* | q.s. |

| **Extragranular phase** | |
|---|---|
| Lactose monohydrate | 120.00 mg |
| Colloidal anhydrous silica | 4.00 mg |
| Magnesium stearate | 18.00 mg |

| | |
|---|---|
| * not present in the final product, evaporates during drying | |

**Composition of the amlodipine layer for Examples 15a and 15b**

| **Composition** | **Example 15a** | **Example 15b** |
|---|---|---|
| Amlodipine besylate | 6.94 mg* | 13.88 mg** |
| Dibasic anhydrous calcium phosphate¹⁾ | 144.36 mg | 137.42 mg |
| Croscarmelose sodium | 4.00 mg | 4.00 mg |
| Colloidal anhydrous silica | 1.10 mg | 1.10 mg |
| Magnesium stearate | 3.60 mg | 3.60 mg |

| | | |
|---|---|---|
| * corresponding to 5.00 mg of amlodipine ** corresponding to 10.00 mg of amlodipine ¹⁾ commercially available as Di-Cafos A | | |

The weight of the valsartan layer was 640.00 mg and of the amlodipine layer 160.00 mg. The total weight of the final bilayer tablet was 800.00 mg. The bilayer tablets are optionally coated, using coating layer as described in Examples 1 or 4.

### Example 16a-b

The process of preparation of the granulate of valsartan layer is the same as in Example 4. To the valsartan granulate, colloidal anhydrous silica and magnesium stearate are added to prepare the compression mixture for the valsartan layer.
The amlodipine layer is prepared as direct compression mixture.
The compositions for the double-layered tablets are presented in the table below. In Examples 16a-c, the valsartan layer is the same as described in the table below.

**Composition of the valsartan layer**

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 2.50 mg |
| Purified water* | q.s. |

| **Extragranular phase** | |
|---|---|
| Colloidal anhydrous silica | 1.00 mg |
| Magnesium stearate | 6.50 mg |

| | |
|---|---|
| * not present in the final product, evaporates during drying | |

**Composition of the amlodipine layer for Examples 16a-c**

| **Composition** | **Example 16a** | **Example 16b** | **Example 16c** |
|---|---|---|---|
| Amlodipine besylate | 13.88 mg | 13.88 mg | 13.88 mg |
| Lactose monohydrate | 46.12 mg | / | / |
| Mannitol | / | 46.12 mg | / |
| Dibasic anhydrous calcium phosphate | / | / | / |
| Croscarmelose sodium | 1.50 mg | 1.50 mg | 1.50 mg |
| Colloidal anhydrous silica | 1.00 mg | 1.00 mg | 1.00 mg |
| Magnesium stearate | 2.50 mg | 2.50 mg | 2.50 mg |

The weight of the valsartan layer was 255.00 mg and of the amlodipine layer 65.00 mg. The total weight of the final bilayer tablet was 320.00 mg. The hardness of the cores was about 100 N and disintegration time was 50-60 seconds. The bilayer tablets are optionally coated, using coating layer as described in Examples 1 or 4.

### Example 17

4.00 kg of valsartan, 2.05 kg of microcrystalline cellulose (commercially available as Avicel PH101) and 62.5 g of croscarmellose sodium were homogenized in a high-shear mixer Collette Gral 75.

75 g of povidone was dissolved in 3.00 kg of purified water was sprayed into the mixture of valsartan and excipients as listed above. The granulate obtained was dried in a fluid-bed dryer Glatt WSg 5 in 2 steps as described in Example 4. The LOD of the granulate was 1.23% (performed at 85°C for 20 minutes) and 1.17% (performed at 105°C for 5 minutes).

1.50 kg of lactose monohydrate (commercially available as Flowlac 100), 37.5 g of croscarmellose sodium, 50 g of colloidal anhydrous silica and 225 g of magnesium stearate were added to the granulate above and mixed in a high-shear mixer. The LOD of the compression mixture was 1.27% (performed at 85°C for 20 minutes) and 1.21% (performed at 105°C for 5 minutes). The compression mixture was compressed into cores with a theoretical weight of 640.00 mg. Oval punches used have lenght 16 mm and width 8.5 mm with a score. Hardness of the cores was 92-113 N at main pressure of tabletting 6.0 kN and disintegration time was 35 seconds.
Cores were coated as described in Example 4.

### Example 18

The composition of the valsartan granulate and the process of preparation and drying of the granulate was the same as described above in Example 1. Granulating liquid used in the preparation of the granulate was a solution of ethanol and purified water. The extragranular diluent was mannitol.

| **Composition** | |
|---|---|
| **Granulate** | |
| Valsartan | 160.00 mg |
| Microcrystalline cellulose | 82.00 mg |
| Povidone | 3.00 mg |
| Croscarmellose sodium | 4.75 mg |
| Purified water* | q.s. |
| Ethanol* | q.s. |

| **Extragranular phase** | |
|---|---|
| Amlodipine besylate | 13.88 mg |
| Mannitol¹⁾ | 45.37 mg |
| Colloidal anhydrous silica | 2.00 mg |
| Magnesium stearate | 9.00 mg |
| Hardness of the cores | 98-122 N |
| Disintegration time of the cores | 60-70 s |
| Dimensions of the punches | Oval, lenght 13 mm, width 8 mm |
| Main pressure at tabletting | 6.4 kN |

| | |
|---|---|
| * not present in the final product, evaporates during drying ¹⁾ commercially available as Partec M200 | |

### Example 19

48.00 kg of valsartan, 24.6 kg of microcrystalline cellulose (commercially available as Avicel PH200), 0.9 kg of povidone and 0.75 kg of croscarmellose sodium were homogenized in a high-shear mixer Collette Gral 300.

36.00 kg of purified water was sprayed into the mixture of valsartan and excipients as listed above. The granulate obtained was dried in a fluid-bed dryer Glatt 120 at inlet air temperature 25°C at air-flow 2500 m³/h up to the temperature of the granulate 22.2°C for 2 hours 52 minutes. The absolute humidity of the inlet air during drying of the granulate was 0.2-0.7 g/kg.
The wetted granulate was analysed after addition of purified water (initial (t=0)-before beginning of drying) through the whole process of drying.

| **Sample** | **LOD 85°C, 20 min** | **LOD 105°C, 5 min** | **Inlet air temperature** | **Temperature of product** | **Abs.humidity of inlet air** |
|---|---|---|---|---|---|
| Initial (t=0) | 23.82% | 9.36% | 30.2°C | 15.1°C | 0.7 g/kg |
| t=1 h | 11.08% | 5.36% | 21.8°C | 8.3°C | 0.3 g/kg |
| t=2 h | / | 1.68% | 21.5°C | 18.0°C | 0.2 g/kg |
| t=3h | 1.18% | 0.96% | 30.0°C | 22.2°C | 0.3 g/kg |

| | | | | | |
|---|---|---|---|---|---|
| / not measured | | | | | |

After the first drying stepe step 2 followed subsequently to step 1 at an absolute humidity of the inlet air 1.28 g/kg and inlet air temperature 50°C (set-point) for 5 minutes.

18.00 kg of lactose monohydrate (commercially available as Tablettose 70), 0.45 kg of croscarmellose sodium, 0.6 g of colloidal anhydrous silica and 2.7 g of magnesium stearate were added to the granulate above and mixed in a high-shear mixer. The LOD of the compression mixture was 1.23 % (performed at 85°C for 20 minutes) and 1.30 % (performed at 105°C for 5 minutes). The compression mixture was compressed into cores with a theoretical weight of 320.00 mg. Oval punches used have lenght 13.5 mm and width 7 mm. Hardness of the cores was 98-114 N at main pressure of tabletting 6.2 kN and disintegration time was 60 seconds.

Cores were as desribed in Example 4. The theoretical weight of the film-coated tablets was 328.00 mg.

### Results of stress stability testing for Comparative Example 1, Examples 1 and 6a-d

### Chromatographic conditions for related substances:

Column: Gemini C18, 250 x 4,6mm, 5um particles
Detection: UV, 237 nm
Injection: 10ul
Column temperature: 40°C
Sample temperature: 15°C
Flow: 1 mL/min
Mobile phase: gradient elution
A) 0.01 M NaH₂PO₄ pH=3,2 with H₃PO₄
B) ACN

| time | 0 | 40 | 45 | 46 | 50 |
|---|---|---|---|---|---|
| %A | 75 | 40 | 40 | 75 | 75 |
| %B | 25 | 60 | 60 | 25 | 25 |

Solvent: CH₃CN : H2O =4 : 6 (V/V)

Content of impurities are collected in the tables below.

The degradation product Imp 1 at 3.8 min retention time was identified to be 2-(((2'-(1H-tetrazol-5-yl)-[1,1'-biphenyl]-4-yl)methyl)amino)-3-methylbutanoic acid of formula

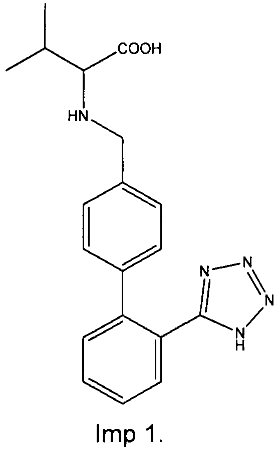

| | **Comparative Example 1** | | | | **Example 1** | | | |
|---|---|---|---|---|---|---|---|---|
| **Condition** | **40°C/75%RH opened** | | **50° closed** | **60° closed** | **40°C/75%RH open** | | **50° closed** | **60° closed** |
| **Time** | **14 days** | **1 month** | **14 days** | **14 days** | **14 days** | **1 month** | **14 days** | **14 days** |
| 3.8 min (Imp 1) | 0.07 | 0.14 | 0.02 | 0.31 | 0.08 | 0.16 | 0.02 | 0.21 |
| 39 min | | | | 0.06 | | | | |
| 43 min | | | | 0.05 | | | | |

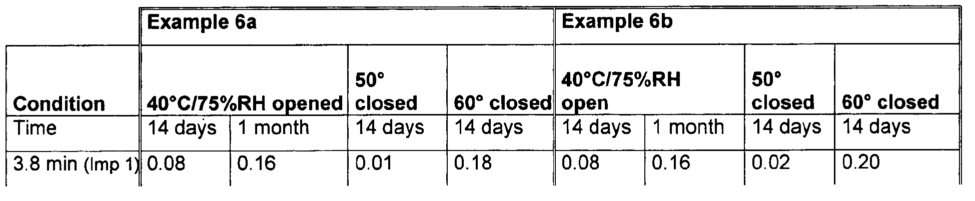

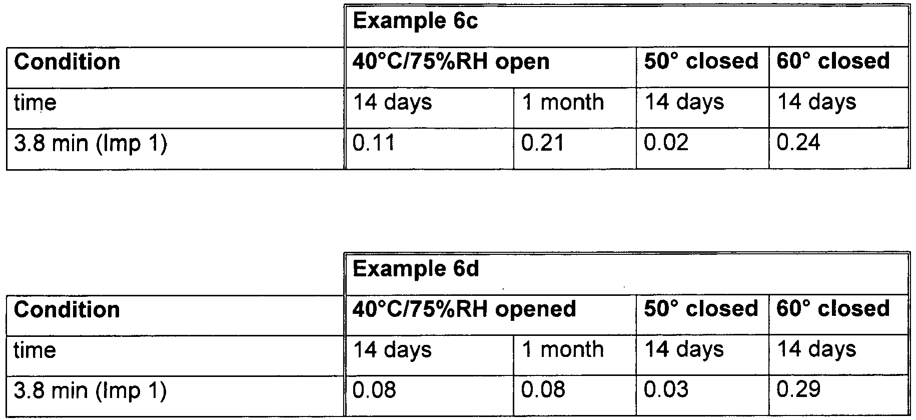

Impurity Imp 1 at retention time 3.8 minutes (the hydrolysed impurity of valsartan of chemical structure C₁₉H₂₁N₅O₂, with exact mass 351.1695; structure is presented above) increases at higher relative humidity (40°C/75%RH opened) and higher temperature (60°C). In Example 2, the Maillard product between amlodipine and lactose was not detected and tablets were not coloured.

## Claims

1. A wet granulation process wherein drying of the granulate comprises two steps at an absolute inlet air humidity of less than 3 g water /kg of air, wherein the first step comprises drying the valsartan comprising granulate at a temperature of 0°C to 40°C for a period of 1 to 7 hours, and the second step comprises drying the valsartan comprising granulate at a temperature of 40°C to 80°C for a period of 1 to 60 minutes.

2. A wet granulation process **characterized in that** drying of the granulate comprises two steps at an absolute inlet air humidity of less than 3 g water/kg of air, and **characterized in that** the wet aqueous granulation process comprises:
(i) preparing a compression mixture by using water or water-based dispersion as granulating liquid to obtain a granulate by
o (a1) granulating a mixture of one or more excipients and the granulating liquid to obtain a granulate, (a2) drying at absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (a3) adding of said valsartan, one or more further pharmaceutically active compounds and optionally further excipient(s) to the granulate to obtain a compression mixture;
(ii) compressing the compression mixture to the desired form, and
(iii) optionaly applying a coating.

3. A process according to claim 1, **characterized in that** the wet aqueous granulation process comprises:
(i) preparing a compression mixture by using water or water-based dispersion as granulating liquid to obtain a granulate by
o (b1) granulating a mixture of one or more excipients, valsartan and the granulating liquid to obtain a granulate, (b2) drying at an absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (b3) adding of one or more further pharmaceutically active compounds and optionally further excipient(s) to the granulate to obtain a compression mixture;
o (c1) granulating a mixture of one or more excipients, a portion of valsartan and the granulating liquid to obtain a granulate, (c2) drying at an absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (c3) adding of the rest of the said valsartan, one or more further pharmaceutically active compounds and optionally further excipient(s) to the granulate to obtain a compression mixture;
(ii) compressing the compression mixture to the desired form, and
(iii) optionaly applying a coating.

4. A process according to claim 2 or 3, **characterized in that** the further pharmaceutically active ingredient is amlodipine.

5. A process according to claim 4, **characterized in that** the further pharmaceutically active ingredient is hydrochlorothiazide.

6. A process according to any of claims 4 and 5, **characterized in that** the further pharmaceutically active ingredients are amlodipine and hydrochlorothiazide.

7. A valsartan comprising granulate, prepared by a wet granulation process wherein drying of the granulate comprises two steps at an absolute inlet air humidity of less than 3 g water/kg of air, wherein the first step comprises drying the valsartan comprising granulate at a temperature of 0°C to 40°C for a period of 1 to 7 hours, and the second step comprises drying the valsartan comprising granulate at a temperature of 40°C to 80°C for a period of 1 to 60 minutes.

8. Use of a valsartan comprising granulate according to claim 7 for the preparation of a valsartan final oral dosage form.

9. Use of a valsartan comprising granulate according to claim 7 for the preparation of a valsartan and amlodipine comprising final oral dosage form.

10. Use of a valsartan comprising granulate according to claim 7 for the preparation of a valsartan and hydrochlorothiazide comprising final oral dosage form.

11. Use of a valsartan comprising granulate according to claim 7 for the preparation of a valsartan, amlodipine and hydrochlorothiazide comprising final oral dosage form.

12. Use of valsartan comprising granulate according to any of claims 9 to 11 for the preparation of a bilayer tablet.

13. Use of valsartan comprising granulate according to claim 11 for the preparation of a trilayer tablet.

14. Compressed compression mixture obtainable by a wet granulation process
wherein drying of the granulate comprises two steps at an absolute inlet air humidity of less than 3 g water/kg of air,
wherein the wet granulation process is a wet aqueous granulation process comprising:
(i) preparing a compression mixture by using water or water-based dispersion as granulating liquid to obtain a granulate by
o (b1) granulating a mixture of one or more excipients, valsartan and the granulating liquid to obtain a granulate, (b2) drying at an absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (b3) adding of one or more further pharmaceutically active compounds and optionally further excipient(s) to the granulate to obtain a compression mixture;
o (c1) granulating a mixture of one or more excipients, a portion of valsartan and the granulating liquid to obtain a granulate, (c2) drying at an absolute inlet air humidity of less than 3 g water /kg of air in two steps, wherein the first step comprises drying at a temperature of 0°C to 40°C for a period of 1 to 7 hours, and the second step comprises drying at a temperature of 40°C to 80°C for a period of 1 to 60 minutes, (c3) adding of the rest of the said valsartan, one or more further pharmaceutically active compounds and optionally further excipient(s) to the granulate to obtain a compression mixture;
(ii) compressing the compression mixture to the desired form, and
(iii) optionaly applying a coating.

15. Compressed compression mixture according to claim 14, which compressed compression mixture is a tablet, which tablet is optionally coated.

## Patentansprüche

1. Feuchtgranulierungsverfahren, wobei das Trocknen des Granulats zwei Schritte bei einer absoluten Einlassluftfeuchtigkeit von unter 3 g Wasser/kg Luft umfasst, wobei der erste Schritt das Trocknen des Valsartan aufweisenden Granulats bei einer Temperatur von 0°C bis 40°C über einen Zeitraum von 1 bis 7 Stunden umfasst und der zweite Schritt das Trocknen des Valsartan umfassenden Granulats bei einer Temperatur von 40°C bis 80°C über einen Zeitraum von 1 bis 60 Minuten umfasst.

2. Feuchtgranulierungsverfahren, **dadurch gekennzeichnet, dass** das Trocknen des Granulats zwei Schritte bei einer absoluten Einlassluftfeuchtigkeit von unter 3 g Wasser/kg Luft umfasst, und **dadurch gekennzeichnet, dass** das wässrige Feuchtgranulierungsverfahren umfasst:
(i) Herstellen eines Kompressionsgemischs unter Verwendung von Wasser oder einer Dispersion auf Wasserbasis als Granulierflüssigkeit zum Erhalten eines Granulats durch
(a1) Granulieren eines Gemischs aus einem oder mehreren Exzipienten und der Granulierflüssigkeit, um ein Granulat zu erhalten, (a2) Trocknen bei einer absoluten Einlassluftfeuchtigkeit von unter 3 g Wasser/kg Luft in zwei Schritten, wobei der erste Schritt das Trocknen bei einer Temperatur von 0°C bis 40°C über einen Zeitraum von 1 bis 7 Stunden umfasst und der zweite Schritt das Trocknen bei einer Temperatur von 40°C bis 80°C über einen Zeitraum von 1 bis 60 Minuten umfasst, (a3) Zugeben des Valsartans, einer oder mehrerer weiterer pharmazeutisch aktiver Verbindungen und wahlweise eines weiteren/weiterer Exzipienten zu dem Granulat, um ein Kompressionsgemisch zu erhalten;
(ii) Komprimieren des Kompressionsgemischs in die gewünschte Form, und
(iii) wahlweises Aufbringen eines Überzugs.

3. Verfahren nach Anspruch 1, **dadurch gekennzeichnet, dass** das wässrige Feuchtgranulierungsverfahren umfasst:
(i) Herstellen eines Kompressionsgemischs unter Verwendung von Wasser oder einer Dispersion auf Wasserbasis als Granulierflüssigkeit zum Erhalten eines Granulats durch
(b1) Granulieren eines Gemischs aus einem oder mehreren Exzipienten, Valsartan und der Granulierflüssigkeit, um ein Granulat zu erhalten, (b2) Trocknen bei einer absoluten Einlassluftfeuchtigkeit von unter 3 g Wasser/kg Luft in zwei Schritten, wobei der erste Schritt das Trocknen bei einer Temperatur von 0°C bis 40°C über einen Zeitraum von 1 bis 7 Stunden umfasst und der zweite Schritt das Trocknen bei einer Temperatur von 40°C bis 80°C über einen Zeitraum von 1 bis 60 Minuten umfasst, (b3) Zugeben von einer oder mehrerer weiterer pharmazeutisch aktiver Verbindungen und wahlweise eines weiteren/weiterer Exzipienten zu dem Granulat, um ein Kompressionsgemisch zu erhalten;
(c1) Granulieren eines Gemischs aus einem oder mehreren Exzipienten, einem Valsartananteil und der Granulierflüssigkeit, um ein Granulat zu erhalten, (c2) Trocknen bei einer absoluten Einlassluftfeuchtigkeit von unter 3 g Wasser/kg Luft in zwei Schritten, wobei der erste Schritt das Trocknen bei einer Temperatur von 0°C bis 40°C über einen Zeitraum von 1 bis 7 Stunden umfasst und der zweite Schritt das Trocknen bei einer Temperatur von 40°C bis 80°C über einen Zeitraum von 1 bis 60 Minuten umfasst, (c3) Zugeben des restlichen Valsartans, einer oder mehrerer weiterer pharmazeutisch aktiver Verbindungen und wahlweise eines weiteren/weiterer Exzipienten zu dem Granulat, um ein Kompressionsgemisch zu erhalten;
(ii) Komprimieren des Kompressionsgemischs in die gewünschte Form, und
(iii) wahlweises Aufbringen eines Überzugs.

4. Verfahren nach Anspruch 2 oder 3, **dadurch gekennzeichnet, dass** der weitere pharmazeutisch aktive Bestandteil Amlodipin ist.

5. Verfahren nach Anspruch 4, **dadurch gekennzeichnet, dass** der weitere pharmazeutisch aktive Bestandteil Hydrochlorthiazid ist.

6. Verfahren nach einem der Ansprüche 4 und 5, **dadurch gekennzeichnet, dass** die weiteren pharmazeutisch aktiven Bestandteile Amlodipin und Hydrochlorthiazid sind.

7. Valsartan umfassendes Granulat, das mittels eines Feuchtgranulierungsverfahrens hergestellt ist, wobei das Trocknen des Granulats zwei Schritte bei einer absoluten Einlassluftfeuchtigkeit von unter 3 g Wasser/kg Luft umfasst, wobei der erste Schritt das Trocknen des Valsartan umfassenden Granulats bei einer Temperatur von 0°C bis 40°C über einen Zeitraum von 1 bis 7 Stunden umfasst und der zweite Schritt das Trocknen des Valsartan umfassenden Granulats bei einer Temperatur von 40°C bis 80°C über einen Zeitraum von 1 bis 60 Minuten umfasst.

8. Verwendung eines Valsartan umfassenden Granulats nach Anspruch 7 zur Herstellung einer endgültigen oralen Valsartan-Darreichungsform.

9. Verwendung eines Valsartan umfassenden Granulats nach Anspruch 7 zur Herstellung einer Valsartan und Amlodipin umfassenden endgültigen oralen Darreichungsform.

10. Verwendung eines Valsartan umfassenden Granulats nach Anspruch 7 zur Herstellung einer Valsartan und Hydrochlorthiazid umfassenden endgültigen oralen Darreichungsform.

11. Verwendung eines Valsartan umfassenden Granulats nach Anspruch 7 zur Herstellung einer Valsartan, Amlodipin und Hydrochlorthiazid umfassenden endgültigen oralen Darreichungsform.

12. Verwendung eines Valsartan umfassenden Granulats nach einem der Ansprüche 9 bis 11 zur Herstellung einer Zweischicht-Tablette.

13. Verwendung eines Valsartan umfassenden Granulats nach Anspruch 11 zur Herstellung einer Dreischicht-Tablette.

14. Komprimiertes Kompressionsgemisch, erhältlich durch ein Feuchtgranulierungsverfahren, wobei das Trocknen des Granulats zwei Schritte bei einer absoluten Einlassluftfeuchtigkeit von unter 3 g Wasser/kg Luft umfasst,
wobei das Feuchtgranulierungsverfahren ein wässriges Feuchtgranulierungsverfahren ist, umfassend:
(i) Herstellen eines Kompressionsgemischs unter Verwendung von Wasser oder einer Dispersion auf Wasserbasis als Granulierflüssigkeit zum Erhalten eines Granulats durch
(b1) Granulieren eines Gemischs aus einem oder mehreren Exzipienten, Valsartan und der Granulierflüssigkeit, um ein Granulat zu erhalten, (a2) Trocknen bei einer absoluten Einlassluftfeuchtigkeit von unter 3 g Wasser/kg Luft in zwei Schritten, wobei der erste Schritt das Trocknen bei einer Temperatur von 0°C bis 40°C über einen Zeitraum von 1 bis 7 Stunden umfasst und der zweite Schritt das Trocknen bei einer Temperatur von 40°C bis 80°C über einen Zeitraum von 1 bis 60 Minuten umfasst, (a3) Zugeben von einer oder mehrerer weiterer pharmazeutisch aktiver Verbindungen und wahlweise eines weiteren/weiterer Exzipienten zu dem Granulat, um ein Kompressionsgemisch zu erhalten;
(c1) Granulieren eines Gemischs aus einem oder mehrerer Exzipienten, einem Valsartananteil und der Granulierflüssigkeit, um ein Granulat zu erhalten, (c2) Trocknen bei einer absoluten Einlassluftfeuchtigkeit von unter 3 g Wasser/kg Luft in zwei Schritten, wobei der erste Schritt das Trocknen bei einer Temperatur von 0°C bis 40°C über einen Zeitraum von 1 bis 7 Stunden umfasst und der zweite Schritt das Trocknen bei einer Temperatur von 40°C bis 80°C über einen Zeitraum von 1 bis 60 Minuten umfasst, (c3) Zugeben des restlichen Valsartans, einer oder mehrerer weiterer pharmazeutisch aktiver Verbindungen und wahlweise eines weiteren/weiterer Exzipienten zu dem Granulat, um ein Kompressionsgemisch zu erhalten;
(ii) Komprimieren des Kompressionsgemischs in die gewünschte Form, und
(iii) wahlweises Aufbringen eines Überzugs.

15. Komprimiertes Kompressionsgemisch nach Anspruch 14, wobei das komprimierte Kompressionsgemisch eine Tablette ist, wobei die Tablette wahlweise überzogen ist.

## Revendications

1. Procédé de granulation par voie humide, dans lequel le séchage du granulé comprend deux étapes à une humidité absolue de l'air entrant inférieure à 3 g d'eau/kg d'air, où la première étape comprend le séchage du granulé contenant le valsartan à une température allant de 0°C à 40°C pendant une période allant de 1 à 7 heures, et la deuxième étape comprend le séchage du granulé contenant le valsartan à une température allant de 40°C à 80°C pendant une période allant de 1 à 60 minutes.

2. Procédé de granulation par voie humide, **caractérisé en ce que** le séchage du granulé comprend deux étapes à une humidité absolue de l'air entrant inférieure à 3 g d'eau/kg d'air, et **caractérisé en ce que** le procédé de granulation par voie humide aqueuse comprend :
(i) la préparation d'un mélange de compression en utilisant de l'eau ou une dispersion à base d'eau comme liquide de granulation, pour obtenir un granulé par
(a1) granulation d'un mélange d'un ou de plusieurs excipients et du liquide de granulation pour obtenir un granulé, (a2) séchage à une humidité absolue de l'air entrant inférieure à 3 g d'eau/kg d'air en deux étapes, où la première étape comprend le séchage à une température allant de 0°C à 40°C pendant une période allant de 1 à 7 heures, la deuxième étape comprend le séchage à une température allant de 40°C à 80°C pendant une période allant de 1 à 60 minutes, (a3) addition dudit valsartan, d'un ou de plusieurs autres composés pharmaceutiquement actifs et le cas échéant, d'autres excipients au granulé pour obtenir un mélange de compression ;
(ii) compression du mélange de compression en la forme souhaitée, et
(iii) le cas échéant, application d'un enrobage.

3. Procédé selon la revendication 1, **caractérisé en ce que** le procédé de granulation par voie humide aqueuse comprend :
(i) la préparation d'un mélange de compression en utilisant de l'eau ou une dispersion à base d'eau comme liquide de granulation, pour obtenir un granulé par
(b1) granulation d'un mélange d'un ou de plusieurs excipients, du valsartan et du liquide de granulation pour obtenir un granulé, (b2) séchage à une humidité absolue de l'air entrant inférieure à 3 g d'eau/kg d'air en deux étapes, où la première étape comprend le séchage à une température allant de 0°C à 40°C pendant une période allant de 1 à 7 heures, la deuxième étape comprend le séchage à une température allant de 40°C à 80°C pendant une période allant de 1 à 60 minutes, (b3) addition d'un ou de plusieurs autres composés pharmaceutiquement actifs et le cas échéant, d'autres excipients au granulé pour obtenir un mélange de compression ;
(c1) granulation d'un mélange d'un ou de plusieurs excipients, d'une partie du valsartan et du liquide de granulation pour obtenir un granulé, (c2) séchage à une humidité absolue de l'air entrant inférieure à 3 g d'eau/kg d'air en deux étapes, où la première étape comprend le séchage à une température allant de 0°C à 40°C pendant une période allant de 1 à 7 heures, la deuxième étape comprend le séchage à une température allant de 40°C à 80°C pendant une période allant de 1 à 60 minutes, (c3) addition du reste du valsartan, d'un ou de plusieurs autres composés pharmaceutiquement actifs et le cas échéant, d'autres excipients au granulé pour obtenir un mélange de compression ;
(ii) compression du mélange de compression en la forme souhaitée, et
(iii) le cas échéant, application d'un enrobage.

4. Procédé selon la revendication 2 ou 3, **caractérisé en ce que** l'autre ingrédient pharmaceutiquement actif est l'amlodipine.

5. Procédé selon la revendication 4, **caractérisé en ce que** l'autre ingrédient pharmaceutiquement actif est l'hydrochlorothiazide.

6. Procédé selon l'une quelconque des revendications 4 et 5, **caractérisé en ce que** les autres ingrédients pharmaceutiquement actifs sont l'amlodipine et l'hydrochlorothiazide.

7. Granulé contenant le valsartan, préparé par un procédé de granulation par voie humide où le séchage du granulé comprend deux étapes à une humidité absolue de l'air entrant inférieure à 3 g d'eau/kg d'air, où la première étape comprend le séchage du granulé contenant le valsartan à une température allant de 0°C à 40°C pendant une période allant de 1 à 7 heures, la deuxième étape comprend le séchage du granulé contenant le valsartan à une température allant de 40°C à 80°C pendant une période allant de 1 à 60 minutes.

8. Utilisation d'un granulé contenant le valsartan selon la revendication 7 pour la préparation d'une forme de dosage oral finale de valsartan.

9. Utilisation du granulé contenant le valsartan selon la revendication 7 pour la préparation d'une forme de dosage oral finale contenant le valsartan et l'amlodipine.

10. Utilisation du granulé contenant le valsartan selon la revendication 7 pour la préparation d'une forme de dosage oral finale contenant le valsartan et l'hydrochlorothiazide.

11. Utilisation du granulé contenant le valsartan selon la revendication 7 pour la préparation d'une forme de dosage oral finale contenant le valsartan, l'amlodipine et l'hydrochlorothiazide.

12. Utilisation du granulé contenant le valsartan selon l'une quelconque des revendications 9 à 11 pour la préparation d'un comprimé bicouche.

13. Utilisation du granulé contenant le valsartan selon la revendication 11 pour la préparation d'un comprimé tricouche.

14. Mélange de compression compressé pouvant être obtenu par un procédé de granulation par voie humide, où le séchage du comprimé comprend deux étapes à une humidité absolue de l'air entrant inférieure à 3 g d'eau/kg d'air en deux étapes,
où le procédé de granulation par voie humide est un procédé de granulation par voie humide aqueuse comprenant :
(i) la préparation d'un mélange de compression en utilisant de l'eau ou une dispersion à base d'eau comme liquide de granulation, pour obtenir un granulé par
(b1) granulation d'un mélange d'un ou de plusieurs excipients, du valsartan et du liquide de granulation pour obtenir un granulé, (b2) séchage à une humidité absolue de l'air entrant inférieure à 3 g d'eau/kg d'air en deux étapes, où la première étape comprend le séchage à une température allant de 0°C à 40°C pendant une période allant de 1 à 7 heures, et la deuxième étape comprend le séchage à une température allant de 40°C à 80°C pendant une période allant de 1 à 60 minutes, (b3) addition d'un ou de plusieurs autres composés pharmaceutiquement actifs et le cas échéant, d'autres excipients au granulé pour obtenir un mélange de compression ;
(c1) granulation d'un mélange d'un ou de plusieurs excipients, d'une partie du valsartan et du liquide de granulation pour obtenir un granulé, (c2) séchage à une humidité absolue de l'air entrant inférieure à 3 g d'eau/kg d'air en deux étapes, où la première étape comprend le séchage à une température allant de 0°C à 40°C pendant une période allant de 1 à 7 heures, la deuxième étape comprend le séchage à une température allant de 40°C à 80°C pendant une période allant de 1 à 60 minutes, (c3) addition du reste du valsartan, d'un ou de plusieurs autres composés pharmaceutiquement actifs et le cas échéant, d'autres excipients au granulé pour obtenir un mélange de compression ;
(ii) compression du mélange de compression en la forme souhaitée, et
(iii) le cas échéant, application d'un enrobage.

15. Mélange de compression compressé selon la revendication 14, le mélange de compression compressé étant un comprimé, qui est le cas échéant enrobé.
